(19) Europäisches Patentamt European Patent Office Office européen des brevets

(11) **EP 4 603 453 A1**

(12) **EUROPEAN PATENT APPLICATION**
published in accordance with Art. 153(4) EPC

(43) Date of publication:
**20.08.2025 Bulletin 2025/34**

(21) Application number: **23877303.0**

(22) Date of filing: **11.10.2023**

(51) International Patent Classification (IPC):
$C01B\ 33/18^{(2006.01)}$  $A61K\ 8/02^{(2006.01)}$
$A61K\ 8/25^{(2006.01)}$  $A61Q\ 1/12^{(2006.01)}$
$A61Q\ 19/00^{(2006.01)}$

(52) Cooperative Patent Classification (CPC):
**A61K 8/02; A61K 8/25; A61Q 1/12; A61Q 19/00;
C01B 33/18**

(86) International application number:
**PCT/JP2023/036868**

(87) International publication number:
**WO 2024/080300 (18.04.2024 Gazette 2024/16)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.10.2022 JP 2022165215
05.09.2023 JP 2023143327**

(71) Applicants:
• **Jikan Techno, Inc.**
  **Osaka-shi, Osaka 553-0003 (JP)**
• **Kinoshita, Takahiro**
  **Osaka-shi, Osaka 553-0003 (JP)**

(72) Inventor: **KINOSHITA, Takahiro**
**Osaka-shi, Osaka 553-0003 (JP)**

(74) Representative: **Studio Torta S.p.A.**
**Via Viotti, 9**
**10121 Torino (IT)**

(54) **SILICA PRODUCTION METHOD AND COSMETIC PRODUCTION METHOD**

(57) [Object]
The present invention is to provide silica and a manufacturing method of the silica in which manufacturing time can shortened and in which the silica can be formed with high purity by removing impurities, and to provide cosmetics mixed with silica having particle shape and size suitable to the characteristics of the cosmetics.

[Means for Solution]
The method is characterized in comprising the steps of crushing process (S41) in which a plant-base raw material (9) is crushed, rinsing process (S42) in which the plant-base raw material obtained in the crushing process is rinsed with water, dehydration process (S43), to remove liquid contained in the plant-base raw material, as the plant-base raw material is stored in a mesh-shaped dehydration container 15 and is rotated by a rotary dehydration device after the rinsing process, calcination process (S44) in which the plant-base raw material is calcined, and fine crushing process (S45) wherein the silica obtained in the calcination process is finely crushed.

[Figure 5]

## Description

### Technical Field

This invention relates to a method for efficiently producing silica, cosmetics as well, from plant-base raw materials

### Background Technology

Silica, conventionally used as fine-grained silicon dioxide, has conventionally low water absorbency when compared to common powders. To utilize this function, silica is used in cosmetics such as eye shadows and foundations to prevent solidification due to moisture, and is also used in creams and emulsions for stabilization and other purposes. Silicon dioxide has also been used as an anode material in battery materials that uses high-purity silicon.

. Among these silicas, crystalline silica is known to be a toxic substance, while amorphous silica is not designated as a toxic substance and can be used for cosmetics, food (including supplements), construction materials, or agricultural fertilizers and feed (for livestock and pet animals).

For example, Patent Document 1 describes rice husk or rice straw charcoal rich in amorphous silica, which is carbonized by a carbonizing apparatus in which rice husks or rice straw are carbonized in an oxygen-free atmosphere while being stirred, wherein the temperature range for carbonizing rice husks or rice straw in said carbonizing apparatus is 500°C to 700 °C.

Further a known method for manufacturing amorphous silica is characterized in that the rice husk charcoal or rice straw charcoal is stirred with ion-exchanged water in the range of 30 to 100 °C, and amorphous silica contained in the rice husk charcoal or rice straw charcoal is dissolved in the ion-exchanged water and extracted.

### Prior Art Documents

### Patent Documents

Patent Document 1 : Japanese Patent Application Publication (KOKAI) No. 2014-181144

### Summary of the Invention

### Problems To Be Solved By This Invention

In the conventional manufacturing method, however, it is necessary to burn organic materials such as cellulose to extract amorphous silica, but it is difficult to increase the purity because metal ions remain as impurities in the plant. The manufacturing process increases its cost where spending time, so it is necessary to make mass-production of the amorphous silica as short a time as possible using a cost-effective manufacturing method.

In addition, there is a growing demand to replace resin micro-beads with silica. Silica, therefore, has been actively developed for cosmetic applications such as foundations and emulsions.

. This invention is made to solve the above-mentioned problems, and to provide silica manufacturing apparatus and method in which the manufacturing time can be shortened and which the purity or whiteness is high by removing impurities, and to provide a cosmetic product mixed with silica whose particle shape and size are adjusted to suit the characteristics of the cosmetic product.

### Means for Solving the Problems

. A disclosed method includes the steps of: soaking a plant-base raw material is soaked in an acid solution; rinsing the plant-base raw material in which the plant-base raw material is soaked in water, agitated, and water-rinsed; dehydrating, after soaking the plant-base raw material, the water contained in the plant-base raw material in which the plant-base raw material is placed in a dehydration container and rotated by a rotary dehydrator; and calcinating the plant-base raw material obtained upon dehydrating the plant-base raw material.

### Advantages of the Invention

The above features reduce the time, required as in the past, for drying when soaking in a solution or rinsing in water to improve production efficiency, and at the same time, promote decomposition of cellulose and other substances, and make removal of impurities possible. In addition, silica can be produced with increased whiteness ideal for use as a cosmetic material.

**Brief Description of the Drawings**

Fig. 1 shows a process flow of the silica production process shown in Example 1 of Embodiment;
Fig. 2 shows a process flow of the silica production process shown in Example 2 of Embodiment;
Fig. 3 shows a process flow of the silica production process shown in Example 3 of Embodiment;
Fig. 4 shows a process flow of the silica production process shown in Example 4 of Embodiment;
Fig. 5 shows a process flow of the silica production process shown in Example 5 of Embodiment;
Fig. 6 is a profile diagram showing time and temperature of silica manufacturing process shown in Example 6 of Embodiment;
Fig. 7 is a micrograph showing a status of silica particle sizes of Embodiment;
Fig. 8 is a diagram showing test data of cosmetics mixed with silica of Embodiment;
Fig. 9 is a diagram showing test data of cosmetics mixed with silica of Embodiment;
Fig. 10 is a diagram showing test data of cosmetics mixed with silica of Embodiment;
Fig. 11 is a diagram showing test data of cosmetics mixed with silica of Embodiment;
Fig. 12 is a diagram showing test data of cosmetics mixed with silica of Embodiment;
Fig. 13 is a diagram showing test data of cosmetics mixed with silica of Embodiment;
Fig. 14 is a diagram showing test data of cosmetics mixed with silica of Embodiment;
Fig. 15 is a diagram showing test data of cosmetics mixed with silica of Embodiment;
Fig. 16 is a schematic diagram showing a manufacturing apparatus for manufacturing silica of Embodiment;
Fig. 17is a diagram showing a process flow of the manufacturing process of silica production shown in Example 6 of Embodiment;
Fig. 18 is a spectrum diagram of silica of Embodiment by X-ray diffraction method; and
Fig. 19 is a spectrum diagram of silica of Embodiment by X-ray diffraction method.

**Embodiments Carrying Out the Invention**

A method for producing silica and cosmetics added with the silica obtained through the method are described herein in detail with reference to the drawings. Embodiments and drawings described below exemplify some of Embodiments of the invention, and are not to be used for the purpose of limiting these configurations, but may be changed or altered as necessary to the extent as not deviated from the gist of the invention.

Plant-base raw material

Described is plant-base raw material 9, the biomass material from which the silica 10 of Example 1 or Example 2 is produced. This invention uses plant-base raw material 9, which can be food residue or waste, to manufacture the final product, amorphous silica. The plant-base raw material 9 is made from plants, wood, etc. In particular, it is possible to obtain the raw material inexpensively by using waste plant-base raw material 9, such as residues from harvesting plants, as the raw material.

[Table 1]

| Composition table of plant-base raw material | C | N | P | $P_2O_5$ | K | $K_2O$ | Ca | Mg | Na |
|---|---|---|---|---|---|---|---|---|---|
| rice straw | 37.4 | 0.53 | 0.06 | 0.14 | 1.75 | 2.11 | 0.05 | 0.19 | 0.11 |
| wheat straw | 40.3 | 0.67 | 0.08 | 0.18 | 1 | 1.21 | 0.21 | 0.11 | 0.06 |
| barley straw | 41.8 | 0.58 | 0.08 | 0.18 | 1.4 | 1.69 | 0.29 | 0.1 | - |
| rice bran | 40.2 | 1.18 | 0.9 | 2.06 | 1.1 | 1.33 | 0.01 | 0.7 | 0.07 |
| rice husks | 34.6 | 0.32 | 0.03 | 0.05 | 0.31 | 0.37 | 0.01 | 0.07 | 0.13 |
| buckwheat straw | 40.3 | 1.08 | 0.21 | 0.48 | 3.13 | 3.77 | 1.35 | 0.14 | - |
| soybean straw | 44.5 | 1.23 | 0.12 | 0.28 | 0.75 | 0.9 | 1.39 | 0.64 | 0.2 |
| sweet potato vines | 42.7 | 3.74 | 0.22 | 0.5 | 3 | 3.62 | 1 | 0.17 | 0.12 |
| turnip leaves | 39.8 | 3.33 | 0.27 | 0.62 | 4.35 | 5.24 | 1.7 | 0.8 | 0.49 |
| carrot leaves | 41.4 | 2.63 | 0.25 | 0.57 | 4.2 | 5.06 | 0.56 | 0.19 | 0.51 |

(continued)

| Composition table of plant-base raw material | C | N | P | $P_2O_5$ | K | $K_2O$ | Ca | Mg | Na |
|---|---|---|---|---|---|---|---|---|---|
| corn culms | 43.8 | 0.92 | 0.09 | 0.21 | 1.32 | 1.59 | 0.24 | 0.12 | - |
| sugarcane tops | 46.1 | 0.99 | 0.1 | 0.23 | 1.2 | 1.45 | 0.37 | 0.12 | 0.18 |
| coconut shells | 46.2 | 3.86 | 0.69 | 1.58 | 2.69 | 3.24 | 0.21 | 0.3 | 0.04 |
| peanut shells | 51.1 | 0.75 | 0.02 | 0.06 | 0.47 | 0.57 | 0.17 | 0.05 | 0.05 |
| mandarin orange peels | 44.5 | 0.76 | 0.05 | 0.11 | 0.58 | 0.7 | 0.4 | 0.06 | 0.07 |
| red cedar sawdust | 51.1 | 0.07 | - | - | - | - | - | - | - |
| larch bark | 59.6 | 0.06 | - | - | - | - | - | - | - |
| fallen leaves of ginkyo | 50.3 | 0.71 | 0.06 | 0.15 | 0.29 | 0.35 | 1.5 | 0.23 | 0.06 |

Table 1 is a composition table of the plant-base raw materials. Table 1 shows the percentages of the composition rate that make up the raw materials shown on the left most to the right below. For example, rice straw contains 37.4% carbon (C), 0.53% nitrogen (N), 0.06% phosphorus (P), 0.14% phosphoric acid (P2O5), 1.75% potassium (K), 2.11% potash (K2O), 0.05% calcium (Ca), 0.19% magnesium (Mg) and 0.11% sodium (Na).

Here, the plant-origined silicon-containing porous plant-base raw material 9 can be calcined at low temperatures (above 600 °C and below 800 °C) to extract silica in an amorphous state. Many of plant-base raw materials 9 are structured with cells regularly arranged along the axis and thickened with silicic acid deposited on the cell walls.

The silicified cell rows are interspersed with compacted narrow cell rows, and silica with a high specific surface area can be obtained by removing the carbides after carbonization. As mentioned above, plant-base raw materials 9 are suitable if these contain a large amount of silicic acid, more than 13% and less than 35% silicic acid.

Examples of plant-base raw materials 9 with relatively high silicon content are shown in Table 1. In addition to rice straw, wheat straw, barley straw, rice bran, rice husks, buckwheat straw, soybean straw, sweet potato vines, turnip leaves, carrot leaves, corn culms, sugarcane tops, coconut shells, barley husks, cocoa husks, cacao pods, peanut shells, mandarin orange peels, red cedar sawdust, larch bark, and fallen leaves of gingko. Other plants themselves may be used instead of residues.

For example, bamboo is useful because its fibers consist of cellulose, hemicellulose, and lignin, and its minerals consist of iron, magnesium, calcium, manganese, copper, nickel, and other minerals. In addition, silanol groups (Si-OH) are extracted when bamboo or bamboo leaves are calcined, and SiO4 are extracted in the process of calcination.

[Table 2]

| Component composition table of plant-base raw material | | | | | | |
|---|---|---|---|---|---|---|
| moisture | ash | fat | lignin | hemicellulose | cellulose | others |
| 8~10% | 15~18% | 0.1~0.5%| | 18~25% | 16~20% | 30~35% | 5~10% |

[Table 3]

| Inorganic chemical composition table of plant-base raw material (wt%) | | | | | | | |
|---|---|---|---|---|---|---|---|
| $SiO_2$ | $Al_2O_3$ | CaO | $Fe_2O_3$ | $K_2O$ | MgO | MnO | $Na_2O$ |
| 92.14 | 0.04 | 0.48 | 0.03 | 3.2 | 0.16 | 0.18 | 0.09 |

Tables 2 and 3 are component composition tables of the rice husks of the plant-base raw materials 9 in Table 1 above, which is the most suitable plant-base raw material 9 in the method of producing silica in the present invention. Table 2 shows the percentages of the components that make up the raw materials. For example, moisture is 8%-10%, ash is 10%-15%, fat is 0.1%-0.5%, lignin is 18%-25%, hemicellulose is 16%-20%, cellulose is 30%-35%, and others are 5%-10%. Thus, the main components of the organic matter that becomes carbide are lignin, hemicellulose, and cellulose.

Table 3 shows the inorganic chemical composition of the plant-base raw material 9 shown in Table 2. The plant-base raw material 9 shown in Table 2 is 80% by weight organic, such as cellulose, and 20% by weight inorganic. The chemical compositions of the inorganic materials in Table 3 are SiO2 of 92.14% by weight, Al2O3 of 0.04% by weight, CaO of 0.48% by weight, Fe2O3 of 0.03% by weight, K2O of 3.2% by weight, MgO of 0.16% by weight, MnO of 0.18% by weight, and Na2O of 0.09% by weight. Plant-base raw materials 9, such as rice husks shown in Table 2, contain a large amount of silicon dioxide (SiO2) in the inorganic materials.

The following Examples 1 to 5 of this embodiment show the silica production method using rice husks among the plant-base raw materials 9.

(Example 1)

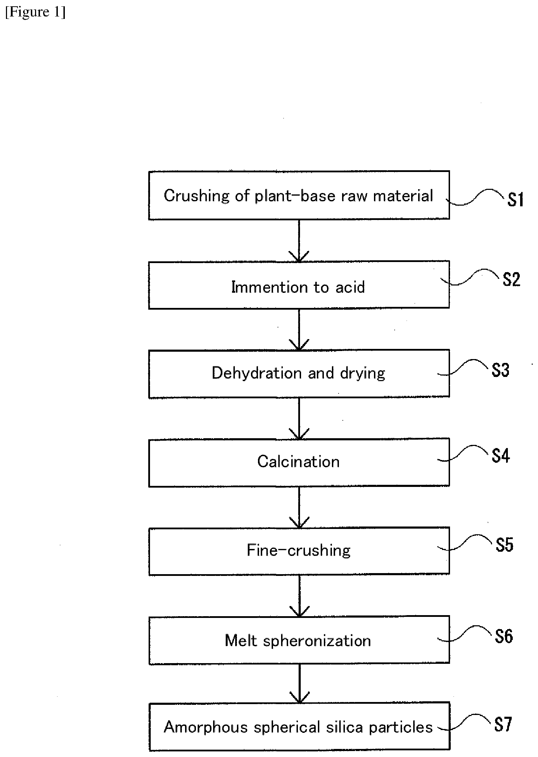

Referring to Fig. 1, a method of producing silica using rice husks among the plant-base raw materials 9 of this embodiment is described as Example 1.

The plant-base raw material 9 is crushed (S1). Since fine particulation can be performed in the process of fine crushing (S5), in this process, it is sufficient to crush the material to the extent that it is soaked inside when immersed in acid, and the size of the material should not slip through the eyes of the dehydration container 15 since the process goes through the dehydration and drying process (S3). The best size after crushing is around 5 to 10 mm. Here, mills, mixers, grinders, and other apparatuses can be used for the crushing method.

Next, the crushed plant-base raw material 9 (S1) is immersed in an acid solution (S2). The acid solution may be formed of sulfuric acid, hydrochloric acid, citric acid, oxalic acid, malic acid, and formic acid. For example, the citric acid solution should be a solution of 1% to 10% by weight citric acid dissolved in pure water (S2). After immersion for about one day, the organic acid and eluted impurities are washed away with pure water or other solution. The solution temperature of the organic acid solution, including citric acid, should be between 20 °C and 80 °C.

Subsequently, the acid-soaked plant-base raw material 9 is placed in a dehydration container 15 and dehydrated with the dehydration container of a rotary dehydrator type as represented by a washing machine (S3). The rotation rate during dehydration should be from 300 to 3000 rpm, preferably with 500 to 1500 rpm.

Dehydration discharges impurities along with water. Especially when citric acid is used, citric acid sequesters metal ions, which are discharged externally as metal chelate compounds (metal complexes) together with the citric acid solution. Dehydration therefore improves the purity of the silica itself after calcination. Next, drying can be done by drying in a dryer, drying in the sun, natural drying, or other methods.

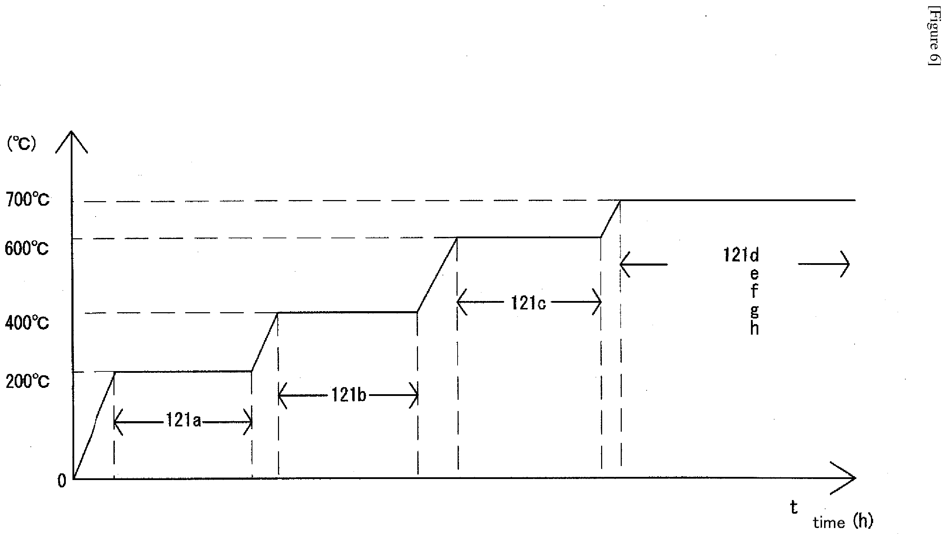

Next, in the calcination process (S4), as shown in Fig. 6, the plant-base raw material 9 is placed in a furnace, and the inside of the furnace is brought to atmospheric pressure where oxygen is supplied. The temperature in the furnace is then raised to 200 °C, and the temperature is maintained at 200 °C for a certain period of time (121a), about 1 to 3 hours.

After that, in a state where oxygen can be supplied, the furnace temperature is raised to 400 °C and the temperature is maintained at 400 °C for a certain period of time (121b), from 1 to 3 hours.

After that, in a state where oxygen can be supplied, the temperature in the furnace is raised to 600 °C and the temperature is maintained at 600 °C for a certain period of time (121c). The temperature is then raised to 700 °C. After the holding time at 700 °C (121d), the rice husks themselves are naturally calcined to make the total calcination time for one day. The best calcination time for 121d, e, f, g, and h may be 10 to 13 hours.

Then, after the calcination is extinguished spontaneously, the calcined silica 10 is removed from the furnace. After the holding time of (121d), the rice husks are calcined by the self-calcination of the rice husks, so after the holding time of (121d), there is no need to use energy, resulting in cost reduction. The purity is improved because the rice husks can be completely calcined by maintaining the temperature at 200 °C and 400 °C for a certain period of time, which requires the most energy when calcining the rice husks.

An example of the composition of silica 10 after calcination is shown below. Silica (SiO2)10 shows 99.1 to 99.2%, while Fe2O3 shows 0.15 to 0.20%, Al2O3 0.05 to 0.03%, K2O 0.05 to 0.08%, CaO 0.2 to 0.5% and MgO 0.02 to 0.065%.

Next, the silica 10 is crushed (S5). The milled silica 10 has a large particle size distribution from 5 to 20 $\mu$m. The milling method may be a jet mill, ball mill, bead mill, and other milling methods.

Next, melt spheronization is performed (S6). In the plasma or gas spraying method, crushed silica powder is fed into a flame at a high temperature of 2000 °C or higher to melt the silica, and the silica melt spherified by surface tension is quenched to obtain spherical silica particles. The resulting spherical silica particles become amorphous because of rapid cooling of the melt silica.

Another spheronization method is the fusion flame process, in which the temperature of the flame treatment is between 1,750 °C and 2,500 °C. Another spheronization method is spray drying.

In plasma melting, a large volume of thermal plasma is generated, and the silica is melted at a high temperature of over 10,000 °C by a high-frequency induction plasma method to produce powder of spherical silica 10 with high sphericity.

The final product is amorphous spherical silica particles 11 (S7) in an amorphous spherical state.

(Example 2)

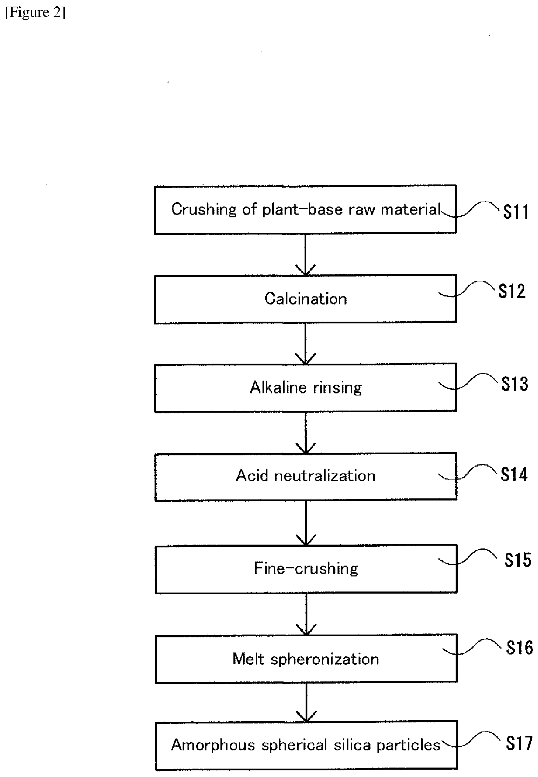

Referring to Fig. 2, a method of producing silica using rice husks among the plant-base raw materials 9 of this embodiment is described as Example 2.

The plant-base raw material 9 is crushed (S11). Since fine particulation is done in fine crushing process (S15), it is sufficient to crush the material to the extent that it is soaked inside when immersed in acid, and the size of the material should not slip through the eyes of the dehydration container 15 since the process goes through the dehydration and drying process. The best size after crushing is around 5 to 10 mm. Here, mills, mixers, grinders, and other apparatuses can be used for the crushing method.

Next, in the calcination process (S12), as shown in Fig. 6, the plant-base raw material 9 is placed in a furnace, and the inside of the furnace is brought to atmospheric pressure and oxygen can be supplied. The temperature in the furnace is then increased to 200 °C, and the temperature is maintained at 200 °C for a certain time (121a) for around 1 to 3 hours.

After that, the furnace temperature is raised to 400 °C in a state where oxygen can be supplied, and the temperature is maintained at 400 °C for a certain period of time (121b), for around 1 to 3 hours.

Subsequently, the temperature in the furnace is raised to 600 °C and the temperature is maintained at 600 °C for a certain period of time (121c), for around 1 to 3 hours. The temperature is then raised to 700 °C. After the holding time at 700 °C (121d), the rice husks themselves are naturally calcined to make the total firing time for one day. The best calcination time for 121d, e, f, g, and h is 10 to 13 hours.

Then, after the calcination is extinguished spontaneously, the calcined silica 10 is removed from the furnace. After the holding time 121d, the rice husks are calcined by the self-calcination of the rice husks, so after the holding time 121d, there is no need to use energy, which results in cost reduction. The purity is improved because the rice husks can be completely calcined by maintaining the temperature at 200 °C and 400 °C for a certain period of time, which requires the most energy when calcining the rice husks.

Next, the calcined silica 10 is immersed in an alkaline solution of PH 8-11 (S13). The alkaline solution is a solution of 1% to 10% concentration made by dissolving baking soda, sodium percarbonate, sodium carbonate, or the like in pure water. The solution temperature should be between 20 °C and 80 °C, and the silica 10 is immersed around for 2 hours (S13).

Next, the calcined silica 10 is neutralized by immersing it in an acid solution (S14). Exemplified as acid solutions are sulfuric acid, hydrochloric acid, citric acid, oxalic acid, malic acid, formic acid, and other acids. For example, the citric acid solution is a solution of 1% to 10% by weight. citric acid dissolved in pure water, and after immersion for about one day, the organic acid and eluted impurities are washed away with pure water. The solution temperature of the organic acid solution, including citric acid, should be between 20 °C and 80 °C. After that, the dehydration and drying process (S3) described above may be performed.

The acid-soaked plant-base raw material 9 is placed in a dehydration container 15 and dehydrated in a rotary dehydrator typical of a washing machine (S3). The rotation rate during dehydration should be from 300 to 3000 rpm, with 500 to 1500 rpm being the best.

Dehydration discharges impurities along with water. Especially when citric acid is used, citric acid sequesters metal ions, which are discharged externally as metal chelate compounds (metal complexes) together with the citric acid solution. Therefore, dehydration improves the purity of the silica itself after calcination. Next, drying can be done by drying in a dryer, drying in the sun, natural drying, or other drying methods.

Next, silica 10 is crushed (S15) in the same way as in step S5 described above. The crushed silica 10 has a large particle size distribution from 5 to 20 $\mu$m. The crushing method may be done by jet mill, ball mill, bead mill, or other milling methods.

Next, melt spheronization is performed as in step S6 described above (S16). In the plasma or gas spraying method, crushed silica powder is fed into a flame at a high temperature of 2000 °C or higher to melt the silica, and the melt silica spherified by surface tension is quenched to obtain spherical silica particles. The resulting spherical silica particles become amorphous because of the rapid cooling of the melt silica.

In addition to above method, spheronization of silica 10 by the fusion flame method is also acceptable, and the temperature of the flame treatment is between 1,750 °C and 2,500 °C. As another spheronization method, spray drying may be used.

In plasma melting, a large volume of thermal plasma is generated and melted at a high temperature of over 10000 °C by the high-frequency induction plasma method to produce a spherical powder of silica 10 with high sphericity.

The final product is amorphous spherical silica particles 11 (S17) in an amorphous spherical state.

(Example 3)

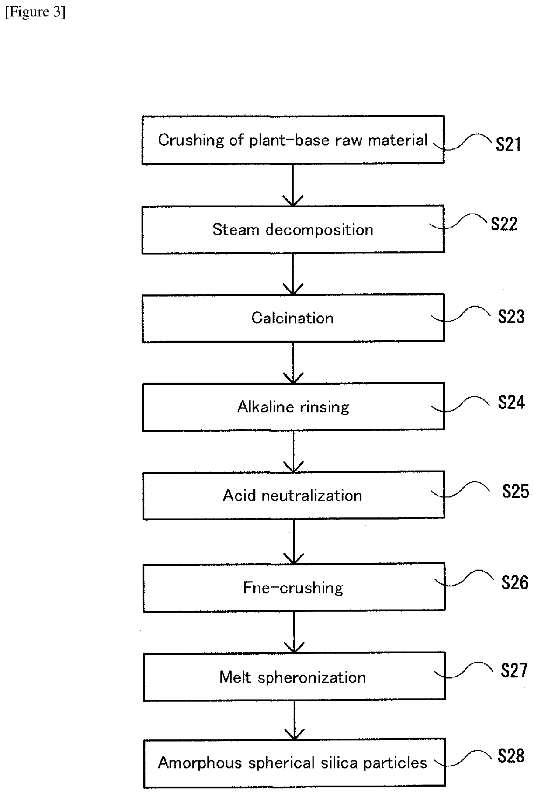

Referring to Fig. 3, a method of producing silica using rice husks among the plant-base raw materials 9 of this embodiment is described in Example 3.

The plant-base raw material 9 is crushed (S21). Since fine particulation is done in the process of fine crushing (S26), in this process, it is sufficient to crush the material to the extent that it is soaked inside when immersed in acid, and the size of the material is large enough not to slip through the eyes of the dehydration container 15 during dehydration and drying. The best size after crushing is 5 to 10 mm. Here, mills, mixers, grinders, and other apparatuses can be used for the crushing method.

Next, the steam decomposition process (S22) is a method to decompose the crushed plant-base raw material 9 by exposing it to steam. This process is especially designed to accelerate the decomposition of lignin and other substances to improve purity and calcination efficiency.

The steam may be pure water or the citric acid solution described above. The steam can be a solution of 1% to 10% by weight. citric acid dissolved in pure water. In the case of steam, the solution is exposed to steam for around 1 to 5 hours to allow the solution to soak into the interior of the plant-base raw material 9. Thereafter, the dehydration and drying process (S3) described above may be followed.

The plant-base raw material 9 soaked in acid is placed in a dehydration container 15 and dehydrated in a rotary dehydrator typical of a washing machine (S3). The rotation rate during dehydration should be from 300 to 3000 rpm, with 500 to 1500 rpm being the best.

Dehydration discharges impurities along with water. Especially when citric acid is used, citric acid sequesters metal ions, which are discharged externally as metal chelate compounds (metal complexes) together with the citric acid solution. Therefore, dehydration improves the purity of the silica itself after calcination. Next, drying can be done by drying in a dryer, drying in the sun, natural drying, or other methods.

Next, in the calcination process (S23), as shown in Fig. 6, the plant-base raw material 9 is placed in a furnace, and the inside of the furnace is brought to atmospheric pressure, where oxygen is supplied. The temperature in the furnace is then raised to 200 °C, and the temperature is maintained at 200 °C for a certain time (121a) for about 1 to 3 hours.

After that, in a state where oxygen can be supplied, the furnace temperature is raised to 400 °C and the temperature is maintained at 400 °C for a certain period of time (121b), around from 1 to 3 hours.

Subsequently, in the state where oxygen can be supplied, the temperature in the furnace is raised to 600 °C and is maintained at 600 °C for a certain period of time (121c) for around 1 to 3 hours. The temperature is then raised to 700 °C. After the holding time at 700 °C (121d), the rice husks themselves are naturally calcined to make the total calcination time for one day. The best calcination time for 121d, e, f, g, and h is 10 to 13 hours.

Subsequently after the calcination is extinguished spontaneously, the calcined silica 10 is removed from the furnace. After the holding time 121d, the rice husk is calcined by self-burning, so there is no need to use energy after the holding time 121d, resulting in cost reduction. The purity is improved because the rice husks can be completely calcined by maintaining the temperature at 200 °C and 400 °C for a certain period of time, which requires the most energy when calcining the rice husks.

Next, the silica 10 is neutralized by immersing it in an acid solution (S25). As acid solutions, exemplified are sulfuric acid, hydrochloric acid, citric acid, oxalic acid, malic acid, formic acid, and other acids. For example, citric acid solution is a solution of 1% to 10% by weight citric acid dissolved in pure water, and after immersion for about one day, the organic acid and eluted impurities are washed away with pure water or the like. The solution temperature of the organic acid solution, including citric acid, should be between 20 °C and 80 °C. After that, the dehydration and drying process (S3) described above may be performed.

The acid-soaked plant-base raw material 9 is placed in a dehydration container 15 and dehydrated in a rotary dehydrator typical of a washing machine (S3). The rotation rate during dehydration should be from 300 to 3000 rpm, with 500 to 1500 rpm being the best.

Dehydration discharges impurities along with water. Especially when citric acid is used, citric acid sequesters metal ions, which are discharged externally as metal chelate compounds (metal complexes) together with the citric acid solution. Therefore, dehydration improves the purity of the silica itself after calcination. Next, drying can be done by drying in a dryer, drying in the sun, natural drying, or other drying methods.

Next, silica 10 is crushed as in step S5 above (S26). The crushed silica 10 has a large particle size distribution from 5 to 20 $\mu$m. The crushing method may be a jet mill, ball mill, bead mill, or other milling apparatuses.

Subsequently, melt spheronization is performed as in step S6 as above described (S27). In the plasma or gas spraying method, crushed silica powder is fed into a flame at a high temperature of 2000 °C or higher to melt the silica, and the silica melt spherified by surface tension is quenched to obtain spherical silica particles. The resulting spherical silica particles are amorphous because of the rapid cooling of the melt silica.

The spheronization of silica 10 by the fusion flame method is also acceptable, and the temperature of the flame treatment is between 1750 °C and 2500 °C. As another method of spheronization, spray-drying may be used.

In plasma melting, a large volume of thermal plasma is generated and melted at a high temperature of over 10000 °C by the high-frequency induction plasma method to produce a spherical powder of silica 10 with high sphericity.

The final product is amorphous spherical silica particles 11 (S28) in an amorphous spherical state.

(Example 4)

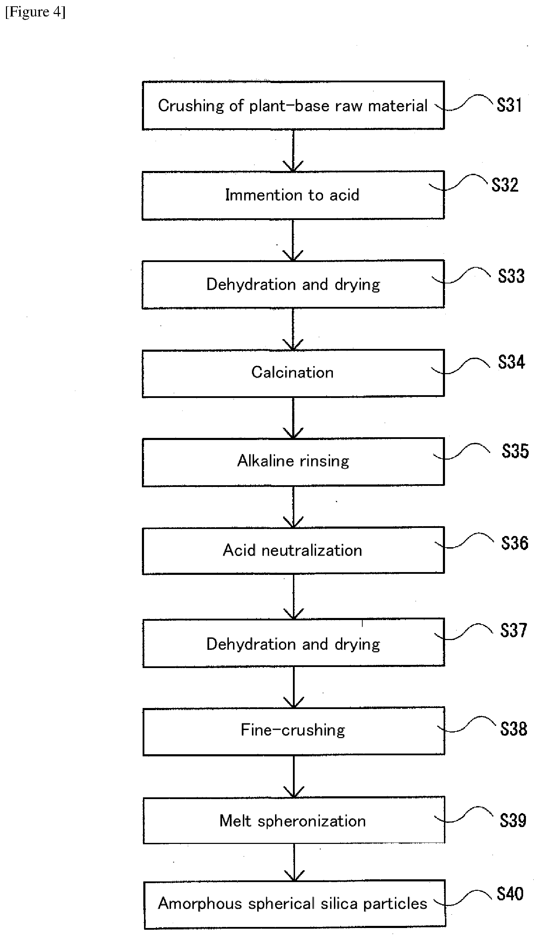

In Example 4, the method of producing silica using rice husks among the plant-base raw materials 9 of this embodiment is described with reference to Fig. 4.

The plant-base raw material 9 is crushed (S31). Since fine particulation is done in the process of fine crushing (S38), in this process, it is sufficient to crush the material to the extent that it is soaked into the acid during soaking, and it is also sufficient that the size does not slip through the eyes of the dehydration container 15 during dehydration and drying. The best size of the crushed material after crushing should be 5 to 10 mm. Here, mills, mixers, grinders, and other apparatuses can be used for the crushing method.

Next, the crushed plant-base raw material 9 (S31) is immersed in an acid solution (S32). As acid solutions exemplified are sulfuric acid, hydrochloric acid, citric acid, oxalic acid, malic acid, formic acid and other acid. For example, the citric acid solution should be a solution of 1% to 10% by weight citric acid dissolved in pure water or the like. After immersion for about one day, the organic acid and eluted impurities are washed away with such as pure water. The solution temperature of the organic acid solution, including citric acid, should be between 20 °C and 80 °C.

Next, the acid-soaked plant-base raw material 9 is placed in a dehydration container 15 and dehydrated in a rotary dehydrator typical of a washing machine (S33). The rotation rate during dehydration should be from 300 to 3000 rpm, with 500 to 1500 rpm being the best.

Dehydration discharges impurities along with water. Especially when citric acid is used, citric acid sequesters metal ions, which are discharged externally as metal chelate compounds (metal complexes) together with the citric acid solution. Therefore, dehydration improves the purity of the silica itself after calcination. Next, drying can be done by drying in a dryer, drying in the sun, natural drying, or other methods.

Next, in the calcination process (S34), as shown in Fig. 6, the plant-base raw material 9 is placed in a furnace, and the inside of the furnace is brought to atmospheric pressure where oxygen is supplied. The temperature in the furnace is then increased to 200 °C, and the temperature is maintained at 200 °C for a certain period of time (121a), about 1 to 3 hours.

After that, the furnace temperature is raised to 400 °C as oxygen is supplied, and the temperature is maintained at 400 °C for a certain period of time (121b), ranging around from 1 to 3 hours.

Subsequently, the temperature in the furnace is raised to 600 °C as oxygen is supplied, and the temperature is maintained at 600 °C for a certain period of time (121c). The temperature is then raised to 700 °C. After the holding time at 700 °C (121d), the rice husks themselves are naturally calcined to make the total firing time one day. The best calcining time for 121d, e, f, g, and h is 10 to 13 hours.

Then, after the calcination is extinguished spontaneously, the calcined silica 10 is removed from the furnace. After the holding time 121d, the rice husks are calcined by the self-firing of the rice husks, so after the holding time 121d, there is no need to use energy, which results in cost reduction. The purity is improved because the rice husks can be completely calcined by maintaining the temperature at 200 °C and 400 °C for a certain period of time, which requires the most energy when calcining the rice husks.

Next, the calcined silica 10 is immersed in an alkaline solution of PH 8-11 (S35). The alkaline solution is a 1% to 5% solution of baking soda, sodium percarbonate, sodium carbonate, or the like dissolved in pure water, and the temperature of the solution should be 20 to 80 °C. The silica 10 is soaked for about 2 hours (S35).

Next, the calcined silica 10 is neutralized by immersing it in an acid solution (S36). As acid solutions, exemplified are sulfuric acid, hydrochloric acid, citric acid, oxalic acid, malic acid, formic acid, and other acids. For example, the citric acid solution is a solution of 1% to 10% by weight citric acid dissolved in pure water, and after immersion for about one day, the organic acid and eluted impurities are washed away with pure water. The solution temperature of the organic acid solution, including citric acid, should be between 20 °C and 80 °C.

Next, the acid-soaked plant-base raw material 9 is placed in a dehydration container 15 and dehydrated in a rotary dehydrator typical of a washing machine (S37). The rotation speed during dehydration should be from 300 to 3000 rpm, with 500 to 1500 rpm being the best.

Dehydration discharges impurities along with water. Especially when citric acid is used, citric acid sequesters metal ions, which are discharged externally as metal chelate compounds (metal complexes) together with the citric acid solution. Dehydration therefore improves the purity of the silica itself after calcination. Next, drying can be done by drying in a dryer, drying in the sun, natural drying, or other drying methods.

Next, silica 10 is crushed in the same way as in step S5 above (S38). The crushed silica 10 has many particle size distributions from 5 to 20 $\mu$m. The milling method may be jet mill, ball mill, bead mill, or other milling methods.

Next, melt spheronization is performed as in S6 above (S39). In the plasma or gas spraying method, crushed silica powder is fed into a flame at a high temperature of 2,000 °C or higher to melt the silica, and the silica melt spherified by surface tension is quenched to obtain spherical silica particles. The resulting spherical silica particles are amorphous because of the rapid cooling of the melt silica.

The spheronization of silica 10 by the fusion flame method is also acceptable, where the temperature of the flame treatment is between 1750 °C and 2500 °C. Another spheronization method is spray drying.

In plasma melting, a large volume of thermal plasma is generated and melted at a high temperature of over 10000 °C by the high-frequency induction plasma method to produce a spherical powder of silica 10 with high sphericity.

The final product is amorphous spherical silica particles 11 (S40) in an amorphous spherical state.

(Example 5)

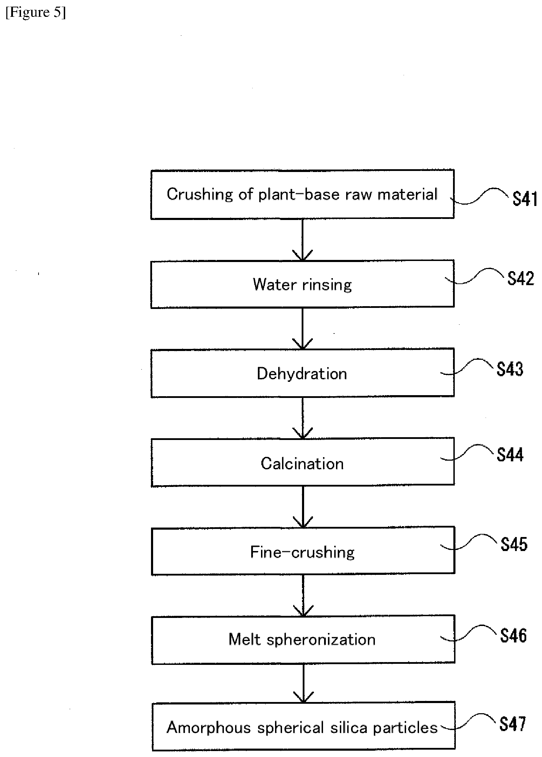

Referring to Fig. 5, a method of producing silica using rice husks among the plant-base raw materials 9 of this embodiment is described in Example 5.

The plant-base raw material 9 is crushed (S41). Since fine crushing is performed in the process of fine crushing step (S45), in this process, it is sufficient to crush the material to the extent that water is soaked into the interior, and the size of the material should not slip through the eyes of the dehydration container 15 since it goes through the dehydration process (S43). The size of the crushed material is best if it is 5 to 10 mm in diameter. Here, mills, mixers, grinders, and other milling apparatuses can be used for the crushing method.

Next, the crushed plant-base raw material 9 (S41) is washed with water (S2). For example, the rice husks are soaked in pure water; after soaking the rice husks for about one day, such as stones, and mud are washed away. The temperature of the liquid should be from room temperature to 80 °C. Washing with water (S2) can be done by pouring water and then agitating and washing. The material can also be washed by agitating while pouring water little by little.

Next, the water-washed plant-base raw material 9 is placed in a dehydration container 15 and dehydrated in a rotary dehydrator typical of a washing machine (S43). The rotation rate during dehydration should be from 300 to 3000 rpm, with 500 to 1500 rpm being the best.

Dehydration discharges impurities along with water. Then, it is possible to move on to the next calcination process without going through the drying process. The dehydration process by rotation also accelerates the decomposition of the rice husk tissues, and it is confirmed that the next process can be proceeded to without any particular drying process. Therefore, it is possible to shorten the production time by reducing the processes.

Accordingly, in Examples 1 through 4, if rotary dehydration is used, it is not necessary to obtain a drying process, but drying reduces the effects of corrosion on the machine.

Next, in the calcination process (S44), as shown in Fig. 6, the plant-base raw material 9 is placed in a furnace, and the inside of the furnace is brought to atmospheric pressure where oxygen can be supplied. The temperature in the furnace is then increased to 200 °C, and the temperature is maintained at 200 °C for a certain time (121a) for about 1 to 3 hours.

Subseqently, the furnace temperature is raised to 400 °C, and the temperature is maintained at 400 °C for a certain period of time (121b), ranging from 1 to 3 hours.

After that, the temperature in the furnace is raised to 600 °C and the temperature is maintained at 600 °C for a certain period of time (121c). The temperature is then raised to 700 °C. After the holding time at 700 °C (121d), the rice husks themselves are naturally calcinated to make the total calcination time one day. The best calcination time for 121d, e, f, g, and h is 10 to 13 hours.

Then, after the calcination is extinguished spontaneously, the calcined silica 10 is removed from the furnace. After the holding time 121d, the rice husks are calcined by the self-firing of the rice husks, so after the holding time 121d, there is no need to use energy, which results in cost reduction. The purity is improved because the rice husks can be completely calcined by maintaining the temperature at 200 °C and 400 °C for a certain period of time, which requires the most energy when calcining the rice husks.

The purity obtained by ICP emission spectrometry of silica 10 produced by the above production method is 97.7% to 98.8%. Other typical metals included are Ca, K, AL, Mg, Mn, Na, P, Zn, and other metals. Ca is from 4700 ppm to 11000 ppm; K is from 75 0ppm to 15000 ppm; Mg is from 730 ppm to 1500 ppm; Mn is from 450 ppm to 640 ppm; P is from 270 ppm to 470 ppm; and Zn is from 89 ppm to 110 ppm.

Next, silica 10 is crushed (S45) as in step S5 above. The crushed silica 10 has more particle size distribution in the size of 5 to 20 $\mu$m. The silica is crushed by jet mills, ball mills, bead mills, and other methods.

Next, melt spheronization is performed as in step S6 above (S46). In the plasma or gas spraying method, crushed silica powder is fed into a flame at a high temperature of 2000 °C or higher to melt the silica, and the silica melt, spherified by surface tension, is quenched to obtain spherical silica particles. The resulting spherical silica particles are amorphous because of the rapid cooling of the melt silica.

The spheronization of silica 10 by the fusion flame method is also acceptable, and the temperature of the flame treatment is between 1750 °C and 2500 °C. Spheronization by spray drying is also acceptable as another spheronization method.

In plasma melting, a large volume of thermal plasma is generated and melted at a high temperature of over 10000 °C by the high-frequency induction plasma method to produce a spherical powder of silica 10 with a high degree of sphericity. In the final stage, amorphous spherical silica particles 11 are produced (S47) in an amorphous spherical state.

(Example 6)

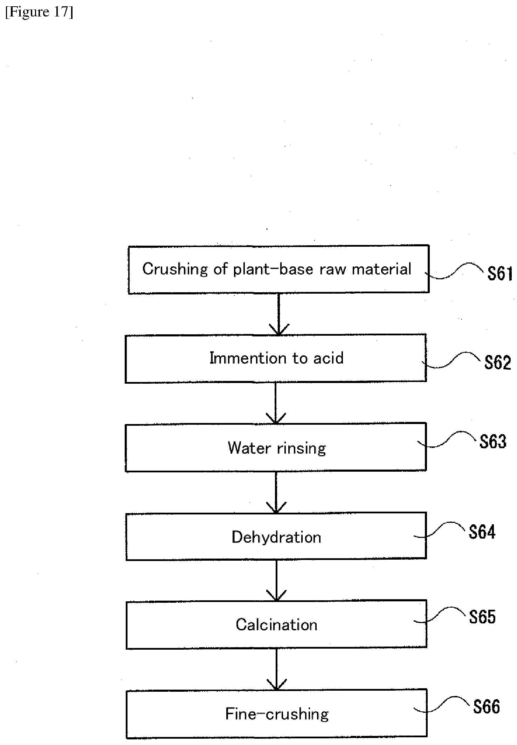

Referring to Fig. 17, a method of producing silica by using rice husks among the plant-base raw materials 9 of this embodiment in Example 6 is described.

The plant-base raw material 9 is crushed (S61). Since fine particulation is done in the process of fine crushing (S66) described below, in this process, it is sufficient to crush the material to the extent that it is soaked into the acid when immersed in the acid, and the size should not slip through the eyes of the dehydration container 15 as it goes through the dehydration process (S64). The best size after crushing is 5 to 10 mm. Here, mills, mixers, grinders, and other apparatuses can be used for the crushing method.

Next, the crushed plant-base raw material 9 is immersed in acid (S62). As acid solutions, exemplified are sulfuric acid, hydrochloric acid, citric acid, oxalic acid, malic acid, formic acid, and other acids. For example, citric acid solution is a solution of 1% to 10% by weight. citric acid dissolved in pure water. After immersion for about one day, the organic acid and eluted impurities are washed away with such as pure water. The solution temperature of the organic acid solution, including citric acid, should be between 20 °C and 80 °C.

Next, rinsing process (S63) for rinsing the material with water is performed. For example, after soaking the rice husks in pure water, the rice husks, stones, mud, etc. are washed away. The liquid temperature should be from room temperature to 80 °C. In the rinsing process (S63), water is poured in and then agitated in a way like a washing machine. Alternatively, the rinsing process can be performed by agitating while pouring the water in little by little. This water rinsing process (S63) removes impurities and promotes decomposition of organic substances by agitation.

Subsequently, the water-rinsed plant-base raw material 9 is placed in the dehydration container 15 and dehydrated in a rotary dehydrator typical of a washing machine (S64). The rotation rate during dehydration should be from 300 to 3000 rpm, with 500 to 1500 rpm being the best.

Dehydration discharges impurities along with water. The process can then be transferred to the next calcination process without going through the drying process. The dehydration process by rotation also accelerates the decomposition of the rice husk tissue, and it is confirmed that the next process can be proceeded to without any particular drying process. Therefore, it is possible to shorten the production time by reducing the number of processes. In addition, it was possible to produce silica in a pure white color compared to products that underwent a drying process.

Therefore, in Examples 1 through 5, it is not necessary to obtain a drying process if rotary dehydration is used, but drying reduces the effects of corrosion and other problems on the machine.

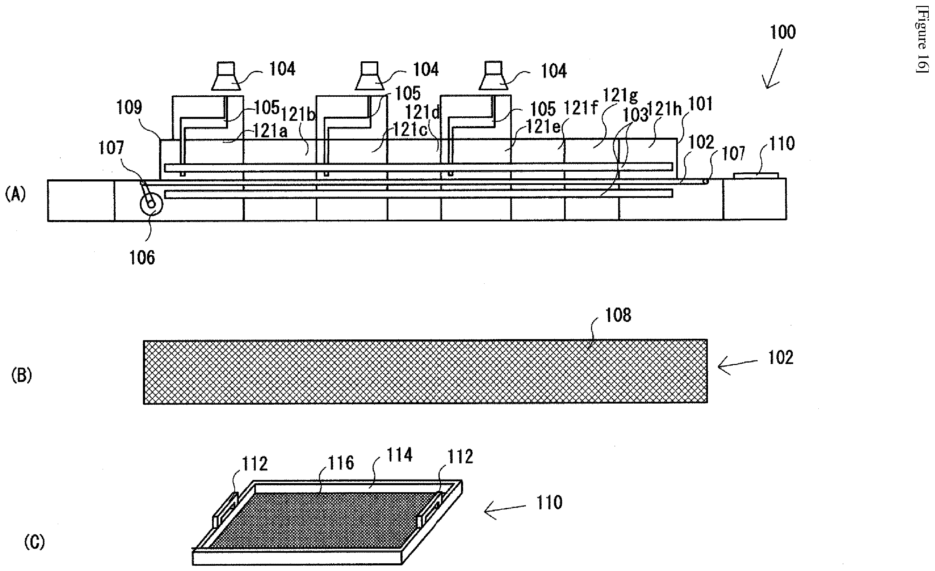

Next, in the calcination process (S65), as shown in Fig. 6 and Fig. 16, the plant-base raw material 9 is placed in a mesh-type continuous calcination furnace 100, as described below. The inside of the furnace is set to atmospheric pressure and oxygen can be supplied, and the temperature in the furnace is increased to 200 °C in the region (121a) to render the material go through the region at the temperature 200 ° C.

After that, the furnace is made to be in a state where oxygen can be supplied, and the material is rendered to pass through the region (121b) at a temperature range where the furnace temperature is increased to 400 °C.

After that, the furnace is made to be in a state where oxygen can be supplied, and the material is rendered to pass through the region (121c) at a temperature range where the furnace temperature is increased to 600 °C.

After that, the furnace is made to be in a state where oxygen can be supplied, the furnace temperature is increased to 700 °C. The material is rendered to pass through the regions (121d, e, f, g, and h) of the temperature range where the furnace temperature is increased to 700 °C. The best time to pass through the regions (121a to h) is from 3 to 10 hours.

After the calcination is extinguished spontaneously, the calcined silica 10 is removed from the furnace. The purity of silica 10 after calcination (h and after) is improved because the silica becomes amorphous by spontaneous quenching and rapid cooling, and organic materials such as cellulose can be calcined completely.

The purity of silica 10 produced by the production methods of Examples 1 to 6 above is 97.7% to 98.8% as determined by ICP emission spectrometry. Other typical metals included are Ca, K, AL, Mg, Mn, Na, P, Zn, and other elements. Ca is from 4700ppm to 11000ppm; K is from 750ppm to 15000ppm; Mg is from 730ppm to 1500ppm; Mn is from 450ppm to 640ppm; P is from 270ppm to 470ppm; and Zn is from 89ppm to 110ppm.

Next, silica 10 is crushed (S66) as in step S5 above. The crushed silica 10 has a particle size distribution more in the size of 5 to 20$\mu$m. The silica is crushed by jet mills, ball mills, bead mills, and other methods.

Subsequently, melt spheronization is performed as in step S6 above (S46). In the plasma or gas spraying method, crushed silica powder is fed into a flame at a high temperature of 2000 °C or higher to melt the silica, and the silica melt spherified by surface tension is quenched to obtain spherical silica particles. The resulting spherical silica particles are amorphous because of the rapid cooling of the melt silica.

In addition, silica 10 can also be spheronized by the fusion flame method, where the temperature of the flame treatment is between 1,750 °C and 2,500 °C. Another spheronization method is spray drying.

In the plasma melting process, a large volume of thermal plasma is generated and melted at a high temperature of over 10000 °C by the high frequency induction plasma method to produce spherical silica 10 powder with a high degree of sphericity. In the final stage, amorphous spherical silica particles 11 can be produced (S47) in an amorphous spherical state.

During the acid immersion (S2), steam decomposition (S22), and water rinsing (S42), the temperature can be set around 120 °C, and the pressure of 80kPa to 150kPa can be applied to these processes. Applying pressure has the effect of shortening the process time and accelerating the decomposition of the tissues.

The dehydration container 15 in Examples 1 to 6 described above can be a metal, cloth, or plastic container with a mesh, slit, or pore structure, or a combination of these structures. The dehydration containers 15 may be bag-shaped, box-shaped, tubular, or the like, as long as the dehydration containers 15 allow water to escape by centrifugal force.

The time distribution between the water rinsing process (S63) and the dehydrating process (S64) is best done at a time ratio of 1:1, and the time distribution of 1:1 to 1:4 is preferably recommended. The dehydration process (S64) reduces the time of the manufacturing process, because by removing only the water, it is possible to move on to the calcination process (S65) even if the product is still somewhat moist. In addition, the removal of impurities by rotation in the dehydration process (S64) is highly effective and also promotes the decomposition of organic tissues.

Another method of dehydration is to place the plant-base raw material 9 in a bag-shaped dehydration container 15 and squeeze the bag with a squeezer to remove water. Agitation in the above-mentioned water rinsing process (S63) and other steps is performed with a vortex or blade type agitator.

The first crushing process (S1, S11, S21, S31, S41, S61) described above need not be provided in particular, and it is sufficient if the material can be finely crushed by the subsequent fine crushing process to the desired size as appropriate.

During the acid immersion step (S2), steam decomposition (S22), and rinsing (S42), the temperature can be set to around 120 °C and pressure of 80kPa to 150kPa can be applied to these processes. Applying pressure has the effect of shortening the process time and accelerating the decomposition of the tissues because there is still a large amount of acid remaining. This shortens the time and improves the pure whiteness of the silica.

In addition, the plant-base raw material 9 after the dehydration process of S43 or S62 in Examples 1 to 6 may not be completely dried, but remains slightly moist before the calcination process (SS44, S65), so the so-called steam activation occurs in the initial state, leading to the destruction of cell walls and the promotion of decomposition. In addition, the generation of micropores by steam activation facilitates the formation of porous materials.

The gravity acceleration of the dehydrator described above in Examples 1 to 6 is preferably from 2G to 6G, and more preferably from 3G to 5G.

(Calcination furnace)

Next, the production apparatus for calcination of silica 10 (S4, S12, S23, S34, S44, S65) is explained with reference to Fig. 16.

Fig. 16 is an overview of the mesh type continuous calcination furnace 100. Fig. 16(a) is an overview view of the internal structure of the mesh-type continuous calcination furnace 100 when viewed from the side. The mesh-type continuous furnace 100 has a drive motor 106 and rollers 107 to which the drive belt is suspended, and has a mesh-type conveyance belt 102. The mesh-type continuous furnace 100 can reach temperatures from room temperature to 1000 °C, and can adjust the temperature gradient and temperature holding time.

In a plurality of divided regions (121a to 121h) of the mesh-type continuous furnace 100, the temperature can be changed in each region. For example, the region 121a is maintained at around 200 °C, the region 121b at around 400 °C, the region 121c at around 600 °C, and the region 121d through 121h at around 700 °C. The time spent in each temperature region is determined by the transport speed of the mesh conveyance belt 102. The best transit time for the regions (121a to 121h) is from 3 to 10 hours.

At the mesh-type continuous calcination furnace 100, plant-base raw material 9 is fed into a plurality of mesh-type storage containers 110 that are conveyed from the entrance 101 through to the exit 109. The mesh type conveyance belt 102 conveys the plurality of mesh type storage containers 110 from the entrance 101 to the exit 109.

Since the plant-base raw material 9 generates gases during burning, the mesh type continuous calcination furnace 100 is equipped with a disposal inlet 105 and an exhaust outlet 104 for making disposal, inside the furnace in consideration of exhausting the gases. This allows combustible gases to be discharged to the outside and reduces the amount of tar remaining in the furnace.

Fig. 16(B) is a plan view of the mesh type conveyance belt 102. The mesh type conveyance belt makes it easier to burn the plant-base raw material 9 by feeding air into the plant-base raw material 9 by making the belt mesh-like.

Fig. 16(c) is an overview of the mesh-type storage container 110. The mesh-type storage container 110 is equipped with a metal mesh 116, made of such as stainless steel, on the bottom of an enclosure 114, which has a certain height, so that the heat from a heater 103 from both the upper and lower sides is easily transferred and air is easily taken in. The mesh storage container 110 is also provided with a handle 112 on the side of the enclosure 114 for easy carrying.

When the plant-base raw material 9 was burned by the burning apparatus as is through a process in which no rinsing or dehydration was performed before any burning was made, crystallization was observed in the silica produced. On the other hand, when the plant-base raw material 9 was dehydrated by rinsing or immersing in acid and then burned, the silica produced was confirmed to be amorphous.

Although described with the mesh-type storage container 110, a container without mesh may be used. In such a case, the thickness of the layer of rice husks to be stored should be reduced in order to increase the volume exposed to air.

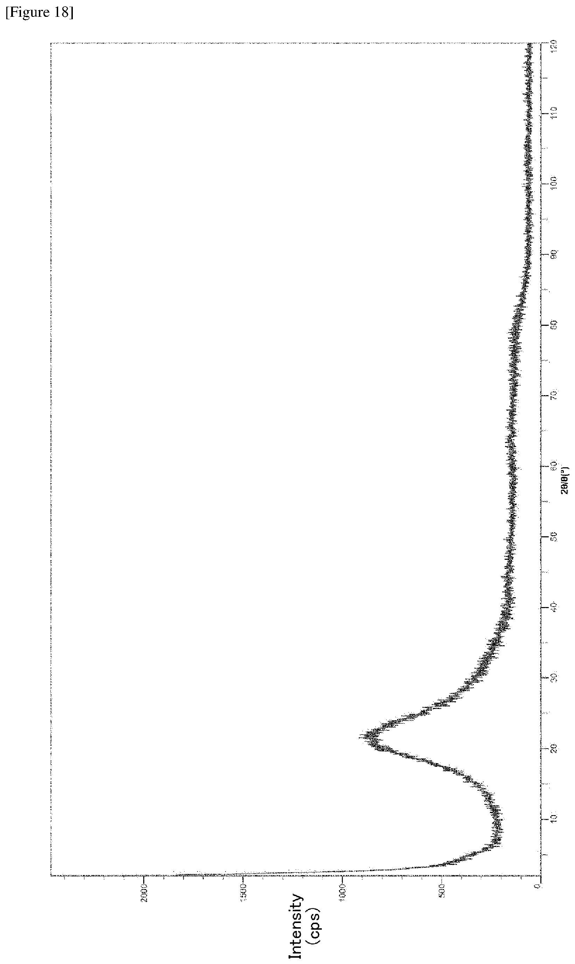

Fig. 18 is a spectral diagram of silica 10 produced by the manufacturing methods of Examples 1, 4, and 6, as measured with an X diffractometer. As shown in Fig. 18, silica 10 is amorphous with a broad peak near 21°.

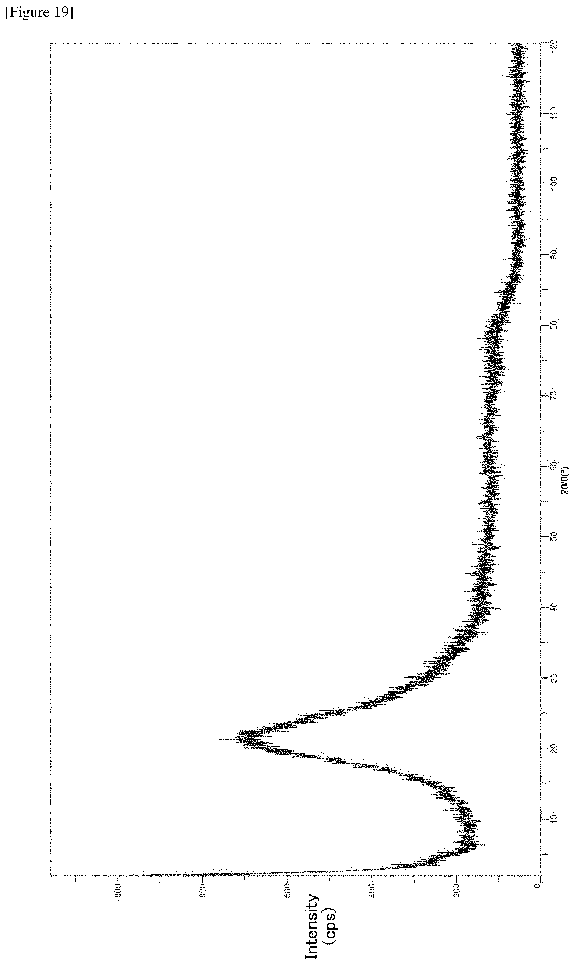

Fig. 19 is a spectral diagram of silica 10 produced by the manufacturing method of Example 5, as measured by an X diffractometer. As shown in Fig. 19, silica 10 was amorphous with a broad peak near 21°.

(Cosmetics)

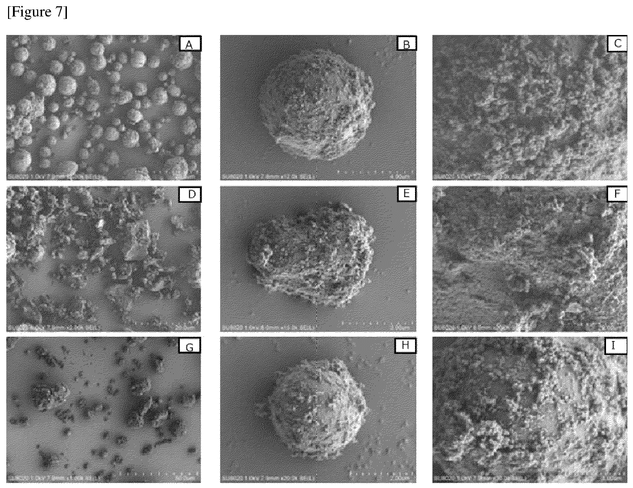

Next, cosmetics 20 using silica 10 or amorphous spherical silica particles 11 produced by the manufacturing methods of Examples 1 to 6 will be described with reference to Table 5 and Figs. 7 to 15.

Fig. 7 shows micrographs of silica 10 or amorphous spherical silica particles 11 produced by the manufacturing method of Examples 1 to 5. Figs. 7(A), 7(B), and 7(C) show amorphous spherical silica particles 11 with particle sizes from 5 to 10 $\mu$m (LU-A10 in the following symbols).

Fig. 7(A) is an overall photograph of a group of amorphous spherical silica particles 11. Fig. 7(B) is an overall photograph of a single particle of amorphous spherical silica particles 11.

Fig. 7(C) is a magnified photograph of a single particle of amorphous spherical silica particles 11. As can be seen in Fig. 7(C), the surface has a crater-like shape with irregular undulations of 0.05 to 0.5 $\mu$m in size throughout variously.

Figs. 7(D), 7(E), and 7(F) show silica 10 produced by the production method of Examples 1 to 5, which only obtained by the fine crushing process (S5, S15, S26, S38, S45, S66) before melt spheronization (S6) or the like. Those are silica 10 with particle sizes from 5 to 10 $\mu$m (LU in the following symbol -C10).

Fig. 7(D) is an overall photograph of a group of silica 10 particles. Fig. 7(E) is an overall photograph of a single particle of silica 10. Fig. 7(F) is a magnified photograph of a single particle of amorphous spherical silica particles 11. As can be seen in Fig. 7(D), the particles vary in shape and size. As can be seen in Fig. 7(E), the particles are blocky, and as can be seen in Fig. 7(F), the particle surfaces are crater-like in shape, varying in size and irregularly formed with undulations of 0.05 to 0.5 $\mu$m.

Figs. 7(G), 7(H), and 7(I) show amorphous spherical silica particles 11 (LU-C5 in the following symbols) with the particle size of 5 $\mu$m or less. Fig. 7(G) is an overall photograph of a group of amorphous spherical silica particles 11. Fig. 7(H) is an overall photograph of a single particle of amorphous spherical silica particles 11. Fig. 7(I) is a magnified photograph of a single particle of amorphous spherical silica particles 11. As can be seen in Fig. 7(I), the surface is crater-like in shape, varying in size and irregularly formed entirely with undulations of 0.01 to 0.1 $\mu$m.

Table 4.

|  | code | particle size | shape | composition |
|---|---|---|---|---|
| 1 | blank | | | |
| 2 | LU-A10 | 5-10 $\mu$m | spherical | silica |
| 3 | LU-C10 | 5-10 $\mu$m | crushed | silica |
| 4 | LU-C5 | less than 5 $\mu$m | spherical | silica |
| 5 | SiO-15 | 15 $\mu$m | true spherical / porous | silica |
| 6 | SiO-16 | 16 $\mu$m | true spherical / porous | silica |
| 7 | KMP 5 | 5 $\mu$m | true spherical / dense | cross-linked polymethylsilsesquioxane |
| 8 | KSP 5 | 5 $\mu$m | true spherical / dense | cross-linked vinyl dimethicone/ methicone silsesquioxane |
| 9 | KSP 12 | 12 $\mu$m | true spherical / dense | cross-linked vinyl dimethicone/ methicone silsesquioxane |
| 10 | Ny 5 | 5 $\mu$m | true spherical / dense | poly amido 12 (PA12) |
| 11 | Ny 10 | 10 $\mu$m | true spherical / dense | poly amido 12 (nylon 12) |
| 12 | Acl 15 | 15$\mu$m | true spherical / dense | polyacrylic ester crosslinked polymer |
| 13 | Acl 30 | 30 $\mu$m | true spherical / dense | acrylic ester crosslinked polymer |
| 14 | PMM 8 | 8 $\mu$m | true spherical / porous | polymethyl methacrylate |
| 15 | TA-25 | 8-10 $\mu$m | true spherical / porous | cellulose acetate |
| 16 | C-25 | 8-10 $\mu$m | true spherical / porous | cellulose |
| 17 | C-25N | 8-10 $\mu$m | true spherical / dense | cellulose |
| 18 | D-5 | 5 $\mu$m | true spherical / dense | cellulose |

(continued)

|  | code | particle size | shape | composition |
|---|---|---|---|---|
| 19 | D-10 | 10 $\mu$m | true spherical / dense | cellulose |
| 20 | D-30 | 30 $\mu$m | true spherical / dense | cellulose |
| 21 | talc | 15 $\mu$m | thin plate | talc |
| 22 | mica | 10 $\mu$m | thin plate | mica |
| 23 | PDM | 6 $\mu$m | thin plate | synthetic mica |

Table 4 shows a table of comparative materials when tested on each of the produced cosmetics 20. LU-A10 is amorphous spherical silica particles 11 with a particle size of 5 to 10 $\mu$m produced by the manufacturing methods of Examples 1 to 5. LU-C10 is silica 10 with a particle size of 5 to 10 $\mu$m produced by the manufacturing methods of Examples 1 to 5. LU-C5 is amorphous spherical silica particles 11 with a particle size of 5 $\mu$m or less produced by the production method of Examples 1 to 5.

SiO-15 is a comparison sample of porous mineral silica truly-spherical particles having the particle size of 15 $\mu$m; SiO-16 is a comparison sample of porous mineral silica truly-spherical particles having the particle size of 16 $\mu$m. KMP5 is a comparison sample of a truly spherical cross-linked polymer of polymethylsilsesquioxane with a dense particle size of 5 $\mu$m; KSP5 is a comparison sample of a truly spherical cross-linked polymer of vinyl dimethicone/methicone silsesquioxane with a dense particle size of 5 $\mu$m; KSP12 is a comparison sample of a vinyl dimethicone/methicone silsesquioxane cross-linked polymer in a truly spherical shape with a dense particle size of 12 $\mu$m.

Ny5 is a comparison sample of polyamide truly spherical particles with a dense 5 $\mu$m particle size; Ny10 is a comparison sample of polyamide truly spherical particles with a dense 10 $\mu$m particle size; Acl15 is a comparison sample of polyacrylic ester crosslinked polymer truly spherical particles with a dense 15 $\mu$m particle size; Acl30 is a comparison sample of a truly spherical cross-linked polymer of acrylics with a dense particle size of 30 $\mu$m; PMM8 is a comparison sample of a truly spherical polymethyl methacrylate with a porous particle size of 8 $\mu$m.

TA-25 is a comparison sample of truly spherical cellulose acetate with porous 8-10 $\mu$m particle size. C-25N is a comparison sample of truly spherical cellulose dense 8-10 $\mu$m particle size. D-5 is a comparison sample of truly spherical cellulose dense 5 $\mu$m particle size. D-10 is a comparison sample of truly spherical cellulose dense 10 $\mu$m particle size. D-30 is a comparison sample of truly spherical cellulose dense 30 $\mu$m particle size. TalcEX-10 is a comparison sample of thin plate shape talc with 15 $\mu$m particle size. MicaY-1800 is a comparison sample of thin plate shape mica with a particle size of 10 $\mu$m. PDM-5L is a comparison sample of thin plate shape synthetic mica with a particle size of 6 $\mu$m.

The following is a description of how the test samples were prepared using the above samples. A dry sample was prepared by mixing each of the above samples and a commercially available foundation at a ratio of 2:18. The oil-added samples were prepared by mixing the above samples and olive oil at a ratio of 2:18. The oils added are not limited to olive oil, but also include jojoba oil, argan oil, rose hip oil, horse oil, sesame oil, macadamia nut oil, avocado oil, coconut oil, marula oil, and other oils.

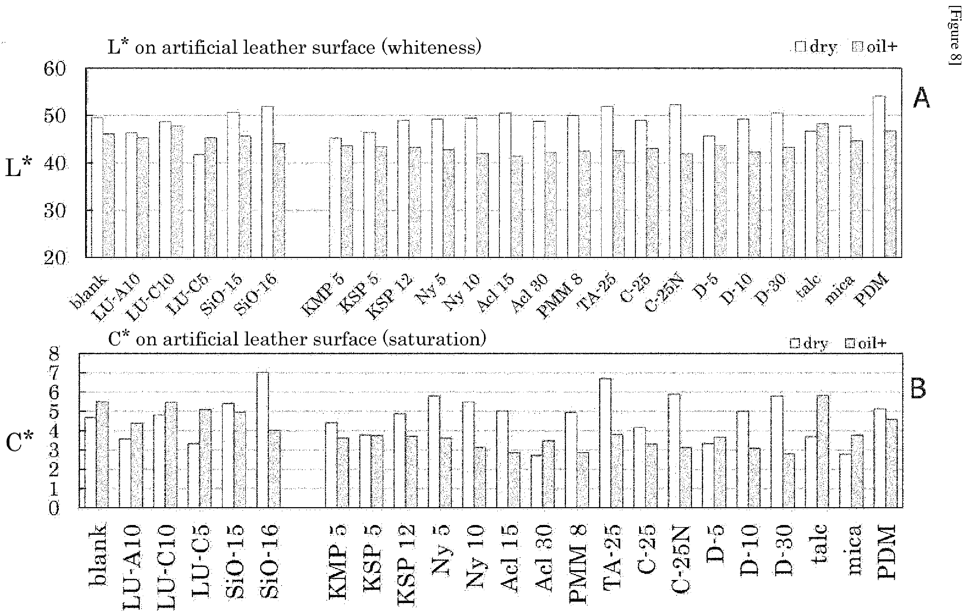

Fig. 8A shows whiteness. White bars in the graph indicate the L* value of the color difference meter when the above dry samples are applied to an artificial leather surface. Filled bars indicate the L* value of the color difference meter when the above oil-added samples are applied to the artificial leather surface. The L* value indicates higher whiteness as larger where black is set to 0 and white is set to 100. The larger the value, the greater the hiding power, and the smaller the value, the higher the transparency.

[Formula 1]

$$C^* = \sqrt{a^{*2} + b^{*2}}$$

Fig. 8B shows saturation. Value C* shows the numerical values according to the calculation formula shown in Formula 1. Value a* shows change in color from red to green, and b* shows change in color from blue to yellow. These values are measured with the color difference meter.

The white bar in the graph shows the C* values when the above dry sample is applied to the artificial leather surface. The filled bars indicate the C* values when the above oil-added sample is applied to the artificial leather surface.

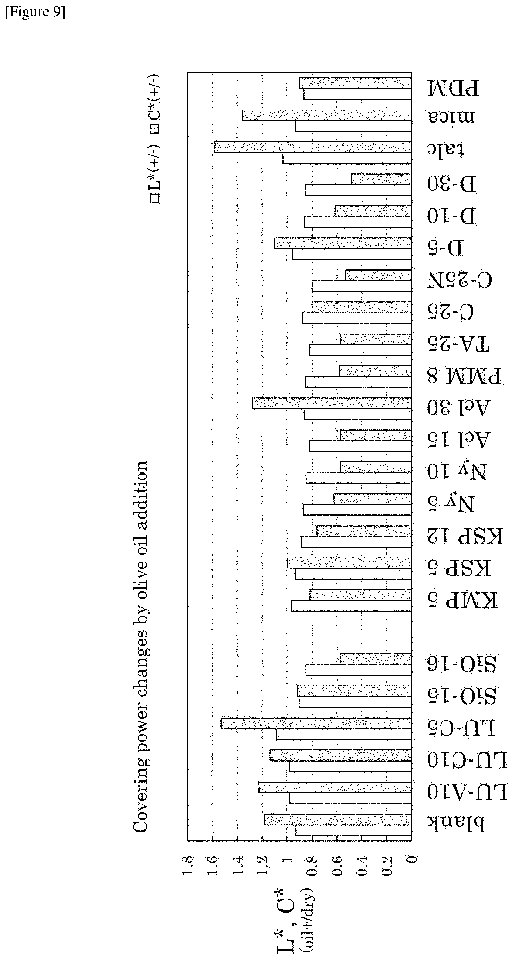

Fig. 9 shows the respective values of L* and C* shown in Fig. 8, the values of the oil-added sample divided by values of the dry sample. The white bars in the graph indicate the L* values. The filled bars indicate the C* values. The value as closer to one has the greater effect of sebum wetting. LU-C10 has the value close to one, so that has characteristics of high coverage and high resistance to sebum wetting.

In accordance with the results above, LU-C10 is evaluated as having characteristics of high coverage and high resistance to sebum wetting.

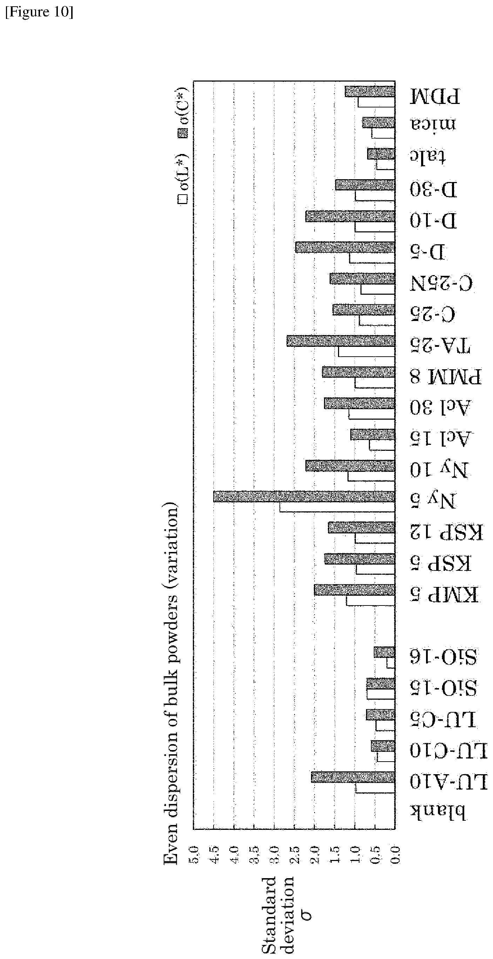

Fig. 10 shows values of a sponge coated with foundation made by the method described above, and the sponge is applied to an artificial leather sheet to evaluate the color dispersion and uniform dispersion. The smaller the value of standard deviation $\alpha$, the more uniform it is. The white bars in the graph show the L* value. The filled bars indicate C* values. The above results confirm that LU-C10 and LU-C5 have small dispersion.

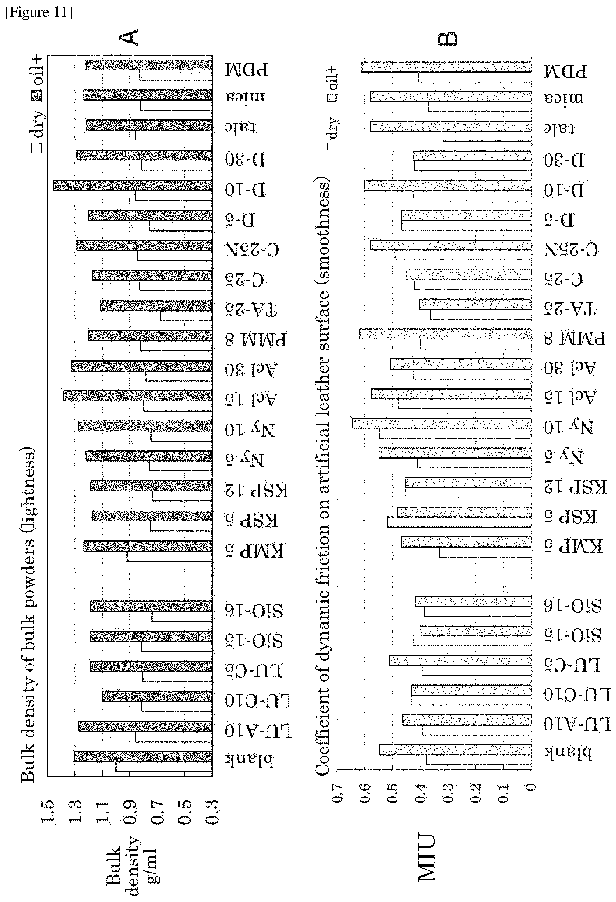

Fig. 11A shows the degree of lightness measured for each bulk density. The white bars in the graph show values for the above dry sample powders. The filled bars indicate values of the powder of the above-mentioned oil-added sample. It was confirmed that LU-C10 has a low bulk density and a low weight.

Fig. 11B shows the coefficient of dynamic friction (MIU) measured by reciprocating the sponge on the surface of the foundation produced by the above preparation method. The white bars in the graph show values of the powder of the above dry sample. The filled bars indicate values of the powder of the above-mentioned oil-added sample. It was confirmed that LU-C10 has the low coefficient of dynamic friction. The smaller this value is, the lighter and smoother the spreading condition is given.

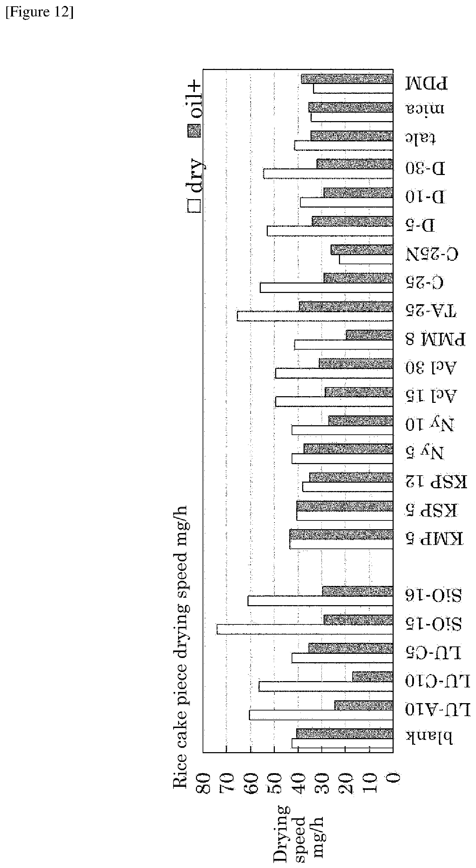

Fig. 12 shows the above drying speed test. A certain amount of the foundation prepared above was applied to an artificial leather-coated rice cake substrate, in which a rice cake piece was covered with oil-absorbent paper thereon and fixed with artificial leather tape on the paper, and the drying speed was measured at room temperature. The values are shown for the above dry sample application. The filled bars indicate the values when the above oil-added sample was applied. It was confirmed that LU-C10 had the low drying speed. The smaller this value is, the higher the moisturizing effect is.

Next, a test with an emulsion is shown. The above comparison sample of various powders 0.2% was added To 10 mL of emulsion dispensed into a test tube, and stirred and mixed to form an emulsion.

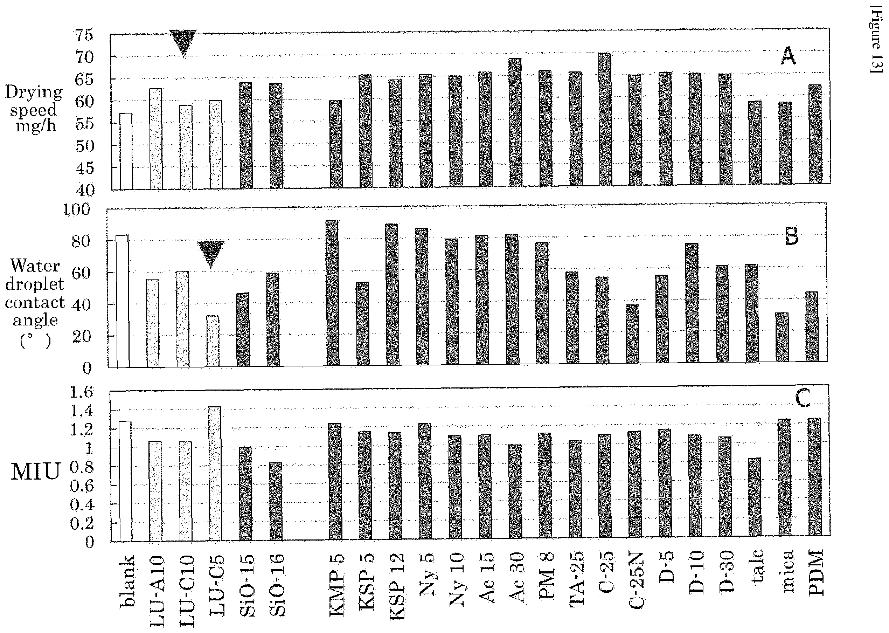

Fig. 13A shows a drying rate test similar to the test described above for the comparison samples formulated with the emulsion. It was confirmed that LU-C10 had a smaller drying rate. The smaller this value is, the higher the moisturizing effect is.

Fig. 13B shows a test in which a certain amount of the comparative sample containing emulsion was added to an artificial leather-coated rice-cake substrate and in which a contact angle was measured using a digital microscope. It was confirmed that LU-C5 had a smaller water droplet contact angle. This low contact angle indicates high wettability; the contact angle of LU-C5 is from above 30 degrees to below 40 degrees.

Fig. 13C shows measured consequences of the coefficient of dynamic friction (MIU) of the comparative sample formulated with emulsion as described above; LU-C5 was confirmed to have a high coefficient of dynamic friction.

As described above, when LU-C5 and LU-C10 are blended in emulsions, the drying rate is low and the moisturizing effect is enhanced. When porous powders are blended, the moisture evaporation effect is generally enhanced, but the amorphous spherical silica particles 11 with the size of 5 $\mu$m or less produced in these Examples have a low contact angle, which increases hydrophilicity and retains moisture. In addition, the particles 11 has the high water retention capacity even after 24 hours, results in the high coefficient of dynamic friction (MIU) on the surface.

Next, in the case of the test with liquid foundation, the above comparison samples of various powders of 0.2% were added to 10 mL of liquid foundation dispensed into a test tube, and were stirred and mixed to produce a liquid foundation.

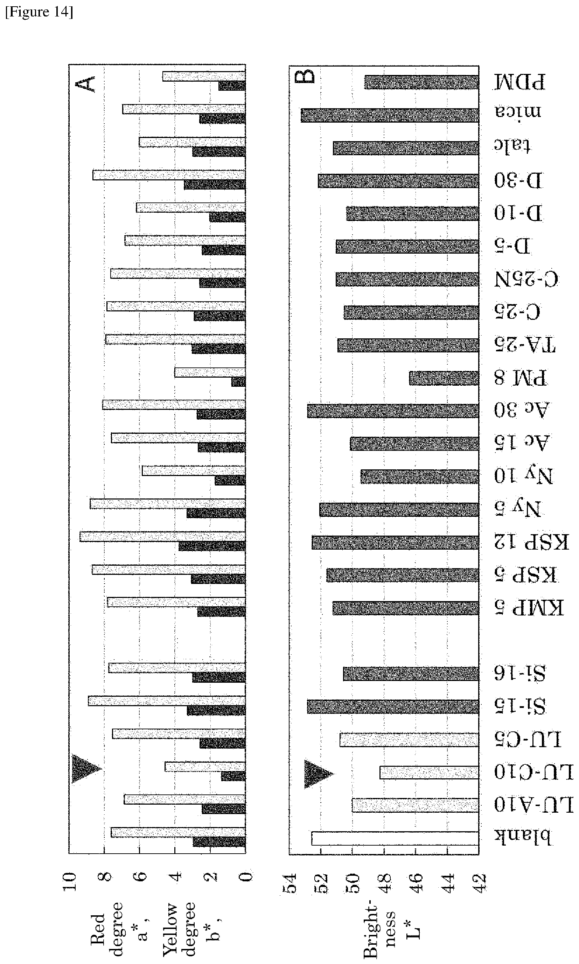

Fig. 14A shows the values measured by a color difference meter after applying the above liquid foundation to an artificial leather surface. Value a* shows a bar of black filled in, indicating a change in color from red to green. Value b* shows a bar of light gray filled in, indicating a change in color from blue to yellow. Transparent feeling can be obtained where values a* and b* are small. LU-C10 shows relatively small values.

Fig. 14B shows the L* values measured by a color difference meter after applying the above liquid foundation to an artificial leather surface. Transparent feeling can be obtained where values a* and b* are small. LU-C10 shows relatively small values.

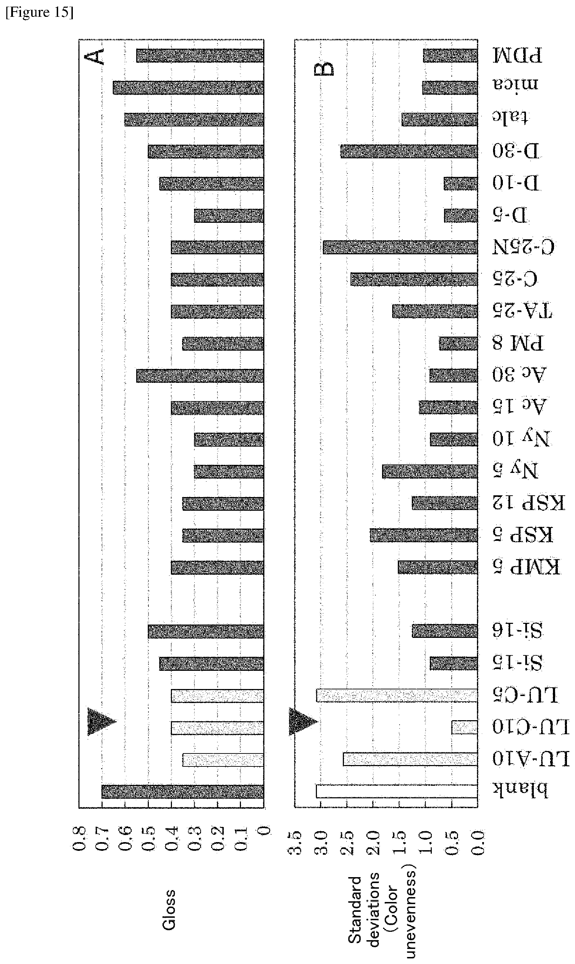

Fig. 15A shows the values measured by a gloss meter after applying the above liquid foundation to an artificial leather surface. LU-A10, LU-C10, and LU-C5 have relatively low values, so that shine is reduced.

Fig. 15B shows the values obtained by applying the above liquid foundation to the surface of artificial leather and measuring the unevenness of color using a color difference meter. LU-C10 particularly shows small values of the standard deviations and high even-dispersion feature.

Blended LU-C10 has extremely low drying speed, high moisturizing effect, low color irregularity, low MIU, and high contact angle, so it spreads smoothly and forms a uniform oily film, resulting in high moisturizing power, and since L*, a*, and b* values are low, resulting in transparent feeling. The Blended LU-C10 has the suppressed gloss, providing a natural transparency with reduced shine and sheen.

(Building Materials)

Silica obtained in the manufacturing processes described above (S5, S15, S26, S38, S45, S66) or subsequently spheroidized by the flame method or other methods can be added to concrete. In this case, the best condition is a particle size of 500 nm and the best condition is spherical.

The specific surface area of silica that underwent the fine crushing process (S5, S15, S26, S38, S45, S66), though not only for building materials, can be ranged from 100 to 300 $m^2/g$.

(Technical Features)

The following are examples of technical features of this embodiment. Those descriptions are not for limiting the technical features but for illustrating exemptions, and also their advantages brought from the technical features are described.

Feature 1

A technical feature includes the steps of:

washing process in which the plant-base raw material is immersed in an aqueous solution for washing, agitated, and rinsed;
dehydrating process dehydrating water contained in the plant-base raw material (S64) after the washing process, the plant-base raw material being stored in a dehydration container and rotated by a rotary dehydrator; and
calcinating process (S65), wherein the plant-base raw material obtained from the dehydration process is calcinated.

The aqueous solution may be an organic acid solution, an alkaline solution, pure water, treated water, or tap water. The best liquid temperature for the aqueous solution is from room temperature (20 °C) to 100 °C. The aqueous solution can be the same type of aqueous solution or a different type of aqueous solution. For example, different types of solutions are acid (organic acid) and water (tap water, treated water or pure water), while the same type of solution is alkaline water and water (tap water, treated water or pure water). The dehydrating container and the container used for rinsing may be the same container. The rinsing process and the dehydrating process may be performed in an apparatus of a type performing with the same device, such as a washing machine.

The above features improve manufacturing efficiency because the time required as in the past for soaking in solution and drying during washing is so reduced, and at the same time, the decomposition of cellulose and other substances is accelerated and impurities can be removed.

Feature 2

The rotation rate of the rotating device is from 300 rpm to 3000 rpm.

The above features improve manufacturing efficiency because the time required as in the past for soaking in solution and drying during washing is so reduced, and at the same time, the decomposition of cellulose and other substances is accelerated and impurities can be removed.

Feature 3

In the calcination process, while oxygen is supplied, the temperature in the furnace is increased to 300 °C and the temperature is maintained at 300 °C for a certain period of time; the temperature in the furnace is then increased to 500 °C, and the temperature is maintained at 500 °C for a certain period of time; the temperature in the furnace is then increased to 700 °C, and the temperature is maintained at 700 °C for a certain period of time; and then the calcination process is done with calcination step by spontaneous calcination of the plant-base raw material itself.

The above features make it possible to calcinate cellulose and other materials efficiently by setting a fixed time at two temperature zones where gas is most easily produced and calories are consumed when cellulose and other materials are burned. In addition, after a certain amount of time is maintained in the two stages, it is possible to burn the plant-base raw material by spontaneous burning without consuming energy.

Feature 4

The process is characterized in that it includes a steam decomposition process (e.g., mainly steam decomposition process (S22)) in which the decomposition of the plant-base raw material is accelerated by steam before the calcination process.

The above features enable the decomposition of cellulose and other materials to be accelerated and burned efficiently.

Feature 5

In amorphous spherical silica particles by fine crushing silica in size from 5 mm to 10 mm and sphering the crushed silica in the silica manufacturing process, silica with a particle diameter of 5 $\mu$m or less, with irregularly blocky in shape, and with irregularly formed undulations of 0.01 to 0.1 $\mu$m on the surface, is added.

The above features make it possible to provide cosmetics with a relatively small gloss value and with reduced shine.

Feature 6

In a group of silicas with a particle size of 5 to 10 $\mu$m, having an irregularly shaped block by the fine crushing process of the silica manufacturing method, some silica with irregularly formed undulations on the surface, is added.

The above features provide high moisturizing effect with extremely low drying speed, low color variation and high contact angle, so that it smoothly spreads and forms a uniform oily film, resulting in high moisturizing power and transparent feeling due to low L*, a*, and b* values. Gloss is also suppressed, making it possible to provide cosmetics with a natural transparency with reduced shine and sheen.

Feature 7

Silica with irregularly formed undulations of 0.05 $\mu$m to 0.5 $\mu$m on the crater-like surface is added.

The above features provide extremely low drying speed, high moisturizing effect, low color irregularity, and high contact angle, resulting in a smooth spreading and uniform oily film, which provides high moisturizing power and transparent feeling. Gloss is also suppressed, making it possible to provide cosmetics with a natural transparency with reduced shine and sheen.

Feature 8

A silica manufacturing apparatus includes; a plurality of storage containers (e.g., mainly mesh-type storage containers 110) in which bottom surfaces are metal mesh for placing the plant-base raw material thereon; a continuous furnace (e.g., mesh-type continuous calcination furnace 100) with a metal mesh-shaped conveyance belt (e.g., mainly mesh-type conveyance belt 102) that conveys the storage containers; a heater member (e.g., mainly heater 103) for burning said plant-base raw material at positions above and below the conveyance belt; an exhaust vent (e.g., mainly exhaust vent 104) for releasing the gases in the continuous furnace during the burning from the inside to the outside of the furnace.

The above features make it possible to provide a silica production layer that enables continuous burning of a burning time and a large number of plant-base raw materials. In addition, the conveyor belts and storage containers are formed with the nets to allow air to be fed into the plant-base raw materials, facilitating the burning of the plant-base raw materials.

Industrial applicability.

The industrial applications of silica include additives for tires, semiconductor materials, electronic equipment materials, and construction materials, in addition to cosmetic materials. Description of the code.

9 Plant-base raw material
10 Silica
11 Amorphous spherical silica particles
15 Dehydration container
100 Mesh type continuous furnace
102 Mesh conveyance belt
103 Heater
104 Exhaust port
110 Mesh storage container
121a, b, c, d, e, f Regions

S41 Crushing process of plant-base raw material
S43 Dehydration process
S44 Calcination process
S5, S15, S26, S38, S45, S66 Fine crushing process.

**Claims**

1. A method for manufacturing silica comprising the steps of:

   rinsing process in which the plant-base raw material is immersed in an aqueous solution, agitated, and rinsed;
   dehydrating process in which the plant-base raw material is stored in a dehydration container after the rinsing process and rotated with a rotary dehydrator to dehydrate liquid contained in the plant-base raw material; and
   calcinating process for calcining the plant-base raw material obtained from the dehydrating process.

2. The method for manufacturing silica according to claim 1, wherein a rotation rate of the rotary dehydrator is from 300 rpm to 3000 rpm.

3. A method for manufacturing cosmetics comprising the steps of:

   rinsing process in which the plant-base raw material is immersed in an aqueous solution, agitated, and rinsed;
   dehydrating process in which the plant-base raw material is stored in a dehydration container after the rinsing process and rotated with a rotary dehydrator to dehydrate liquid contained in the plant-base raw material;
   calcinating process for calcining the plant-base raw material obtained from the dehydrating process;
   fine crushing process for finely crushing the silica obtained through the calcinating process; and
   adding the silica, upon melting the silica obtained through the fine crushing process to make the melt silica spherical.

4. The method for manufacturing cosmetics according to claim 3, wherein the silica is added as having a particle size of 5 to 10 $\mu$m through the fine crushing process with irregularly blocky shape and having irregular undulations on the surface of the silica.

5. The method for manufacturing cosmetics according to claim 4, wherein the silica is added as having irregular undulations of 0.05 to 0.5 $\mu$m on a crater-like surface of the silica.

6. The method for manufacturing silica according to claim 1, wherein the calcinating process is operated with a manufacturing apparatus comprising:

   a plurality of containers for placing the plant-base raw material;
   a continuous furnace having a metal meshed conveyance belt for conveying the containers;
   a heater member for burning the plant-base raw material upon providing plural regions, wherein the burning temperature of the respective regions are changeable; and
   an exhaust vent for releasing gas in the continuous furnace during the burning from the inside to the outside of the furnace.

[Figure 1]

```
┌─────────────────────────────────────────┐
│   Crushing of plant-base raw material    │ ⟿ S1
└─────────────────────────────────────────┘
                    ↓
┌─────────────────────────────────────────┐
│            Immention to acid             │ ⟿ S2
└─────────────────────────────────────────┘
                    ↓
┌─────────────────────────────────────────┐
│          Dehydration and drying          │ ⟿ S3
└─────────────────────────────────────────┘
                    ↓
┌─────────────────────────────────────────┐
│               Calcination                │ ⟿ S4
└─────────────────────────────────────────┘
                    ↓
┌─────────────────────────────────────────┐
│              Fine-crushing               │ ⟿ S5
└─────────────────────────────────────────┘
                    ↓
┌─────────────────────────────────────────┐
│            Melt spheronization           │ ⟿ S6
└─────────────────────────────────────────┘
                    ↓
┌─────────────────────────────────────────┐
│     Amorphous spherical silica particles │ ⟿ S7
└─────────────────────────────────────────┘
```

[Figure 2]

```
┌─────────────────────────────────────────┐
│ Crushing of plant-base raw material      │ ⟶ S11
└─────────────────────────────────────────┘
                    │
                    ▼
┌─────────────────────────────────────────┐
│            Calcination                    │ ⟶ S12
└─────────────────────────────────────────┘
                    │
                    ▼
┌─────────────────────────────────────────┐
│           Alkaline rinsing                │ ⟶ S13
└─────────────────────────────────────────┘
                    │
                    ▼
┌─────────────────────────────────────────┐
│          Acid neutralization              │ ⟶ S14
└─────────────────────────────────────────┘
                    │
                    ▼
┌─────────────────────────────────────────┐
│            Fine-crushing                  │ ⟶ S15
└─────────────────────────────────────────┘
                    │
                    ▼
┌─────────────────────────────────────────┐
│          Melt spheronization              │ ⟶ S16
└─────────────────────────────────────────┘
                    │
                    ▼
┌─────────────────────────────────────────┐
│    Amorphous spherical silica particles   │ ⟶ S17
└─────────────────────────────────────────┘
```

[Figure 3]

```
┌────────────────────────────────────────┐
│  Crushing of plant-base raw material    │──── S21
└────────────────────────────────────────┘
                    │
                    ▼
┌────────────────────────────────────────┐
│           Steam decomposition           │──── S22
└────────────────────────────────────────┘
                    │
                    ▼
┌────────────────────────────────────────┐
│              Calcination                │──── S23
└────────────────────────────────────────┘
                    │
                    ▼
┌────────────────────────────────────────┐
│            Alkaline rinsing             │──── S24
└────────────────────────────────────────┘
                    │
                    ▼
┌────────────────────────────────────────┐
│           Acid neutralization           │──── S25
└────────────────────────────────────────┘
                    │
                    ▼
┌────────────────────────────────────────┐
│             Fne-crushing                │──── S26
└────────────────────────────────────────┘
                    │
                    ▼
┌────────────────────────────────────────┐
│           Melt spheronization           │──── S27
└────────────────────────────────────────┘
                    │
                    ▼
┌────────────────────────────────────────┐
│   Amorphous spherical silica particles  │──── S28
└────────────────────────────────────────┘
```

[Figure 4]

```
┌─────────────────────────────────────┐
│  Crushing of plant-base raw material │ ～ S31
└─────────────────────────────────────┘
                    │
                    ▼
┌─────────────────────────────────────┐
│           Immention to acid          │ ～ S32
└─────────────────────────────────────┘
                    │
                    ▼
┌─────────────────────────────────────┐
│         Dehydration and drying       │ ～ S33
└─────────────────────────────────────┘
                    │
                    ▼
┌─────────────────────────────────────┐
│              Calcination             │ ～ S34
└─────────────────────────────────────┘
                    │
                    ▼
┌─────────────────────────────────────┐
│            Alkaline rinsing          │ ～ S35
└─────────────────────────────────────┘
                    │
                    ▼
┌─────────────────────────────────────┐
│           Acid neutralization        │ ～ S36
└─────────────────────────────────────┘
                    │
                    ▼
┌─────────────────────────────────────┐
│         Dehydration and drying       │ ～ S37
└─────────────────────────────────────┘
                    │
                    ▼
┌─────────────────────────────────────┐
│              Fine-crushing           │ ～ S38
└─────────────────────────────────────┘
                    │
                    ▼
┌─────────────────────────────────────┐
│            Melt spheronization       │ ～ S39
└─────────────────────────────────────┘
                    │
                    ▼
┌─────────────────────────────────────┐
│   Amorphous spherical silica particles │ ～ S40
└─────────────────────────────────────┘
```

[Figure 5]

```
┌─────────────────────────────────────┐
│  Crushing of plant-base raw material │ ⤳ S41
└─────────────────────────────────────┘
                  │
                  ▽
┌─────────────────────────────────────┐
│            Water rinsing             │ ⤳ S42
└─────────────────────────────────────┘
                  │
                  ▽
┌─────────────────────────────────────┐
│             Dehydration              │ ⤳ S43
└─────────────────────────────────────┘
                  │
                  ▽
┌─────────────────────────────────────┐
│             Calcination              │ ⤳ S44
└─────────────────────────────────────┘
                  │
                  ▽
┌─────────────────────────────────────┐
│            Fine-crushing             │ ⤳ S45
└─────────────────────────────────────┘
                  │
                  ▽
┌─────────────────────────────────────┐
│          Melt spheronization         │ ⤳ S46
└─────────────────────────────────────┘
                  │
                  ▽
┌─────────────────────────────────────┐
│  Amorphous spherical silica particles│ ⤳ S47
└─────────────────────────────────────┘
```

[Figure 6]

[Figure 7]

[Figure 8]

EP 4 603 453 A1

L* on artificial leather surface (whiteness)

C* on artificial leather surface (saturation)

[Figure 9]

[Figure 10]

Even dispersion of bulk powders (variation)

Bulk density of bulk powders (lightness)

□ dry  ▨ oil+

Bulk density g/ml

A

Coefficient of dynamic friction on artificial leather surface (smoothness)

□ dry  ▨ oil+

MIU

B

[Figure 11]

EP 4 603 453 A1

[Figure 12]

Rice cake piece drying speed mg/h

[Figure 13]

[Figure 14]

[Figure 15]

[Figure 16]

[Figure 17]

```
┌─────────────────────────────────────────┐
│   Crushing of plant-base raw material    │⌇─── S61
└─────────────────────────────────────────┘
                    │
                    ▼
┌─────────────────────────────────────────┐
│             Immention to acid            │⌇─── S62
└─────────────────────────────────────────┘
                    │
                    ▼
┌─────────────────────────────────────────┐
│              Water rinsing               │⌇─── S63
└─────────────────────────────────────────┘
                    │
                    ▼
┌─────────────────────────────────────────┐
│               Dehydration                │⌇─── S64
└─────────────────────────────────────────┘
                    │
                    ▼
┌─────────────────────────────────────────┐
│               Calcination                │⌇─── S65
└─────────────────────────────────────────┘
                    │
                    ▼
┌─────────────────────────────────────────┐
│              Fine-crushing               │⌇─── S66
└─────────────────────────────────────────┘
```

[Figure 18]

[Figure 19]

## INTERNATIONAL SEARCH REPORT

| | International application No. |
|---|---|
| | **PCT/JP2023/036868** |

| | |
|---|---|
| **A.** | **CLASSIFICATION OF SUBJECT MATTER** |

*C01B 33/18*(2006.01)i; *A61K 8/02*(2006.01)i; *A61K 8/25*(2006.01)i; *A61Q 1/12*(2006.01)i; *A61Q 19/00*(2006.01)i
FI:  C01B33/18 B; A61K8/02; A61K8/25; A61Q1/12; A61Q19/00

According to International Patent Classification (IPC) or to both national classification and IPC

| | |
|---|---|
| **B.** | **FIELDS SEARCHED** |

Minimum documentation searched (classification system followed by classification symbols)

C01B33/00-33/193; A61K8/00-8/99; A61Q1/00-90/00; B09B1/00-5/00; B09C1/00-1/10; F23B10/00-99/00; F27B9/00-9/40

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580/JSTChina (JDreamIII)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | JP 2008-214158 A (MEIWA INDUSTRIAL CO., LTD.) 18 September 2008 (2008-09-18) claims, examples, paragraphs [0001], [0003]-[0013], fig. 1 | 1-2 |
| Y | | 3, 6 |
| Y | JP 62-096310 A (DENKI KAGAKU KOGYO K.K.) 02 May 1987 (1987-05-02) p. 1, lower left column, lines 12-20 | 3 |
| Y | JP 61-072709 A (DENKI KAGAKU KOGYO K.K.) 14 April 1986 (1986-04-14) claims, p. 1, lower left column, lines 10-13, p. 1, lower right column, line 7 to p. 2, upper right column, line 4, examples | 3 |
| Y | JP 2002-114659 A (SHISEIDO CO., LTD.) 16 April 2002 (2002-04-16) paragraph [0014], examples | 3 |
| Y | WO 2021/117442 A1 (SUMITOMO DENKO SHOKETSU GOKIN K.K.) 17 June 2021 (2021-06-17) paragraphs [0010], [0033]-[0068], fig. 1-4 | 6 |
| Y | JP 2004-113938 A (NIPPON CLAYBURN CO., LTD.) 15 April 2004 (2004-04-15) paragraphs [0008]-[0022], fig. 2 | 6 |

☑ Further documents are listed in the continuation of Box C.        ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 December 2023** | **19 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

# EP 4 603 453 A1

## INTERNATIONAL SEARCH REPORT

International application No.

**PCT/JP2023/036868**

### C. DOCUMENTS CONSIDERED TO BE RELEVANT

| Category* | Citation of document, with indication, where appropriate, of the relevant passages | Relevant to claim No. |
|---|---|---|
| Y | CD-ROM of the specification and drawings annexed to the request of Japanese Utility Model Application No. 005811/1992 (Laid-open No. 066054/1993) (DAIHATSU MOTOR CO., LTD.) 31 August 1993 (1993-08-31), paragraphs [0002], [0003], fig. 3 | 6 |
| A | JP 2003-522703 A (RK CHEMICAL CO., LTD.) 29 July 2003 (2003-07-29) entire text, all drawings | 1-6 |
| A | CN 104787770 A (HARBIN INSTITUTE OF TECHNOLOGY) 22 July 2015 (2015-07-22) entire text, all drawings | 1-6 |
| A | JP 2015-504036 A (WUHAN KAIDI GENERAL RESEARCH INSTITUTE OF ENGINEERING & TECHNOLOY CO., LTD.) 05 February 2015 (2015-02-05) entire text, all drawings | 1-6 |
| A | JP 2021-038114 A (JIKAN TECHNO CO., LTD.) 11 March 2021 (2021-03-11) entire text, all drawings | 1-6 |
| A | JP 53-092259 A (WUMIYACK K.K.) 12 August 1978 (1978-08-12) entire text, all drawings | 1-6 |
| A | JP 2017-171515 A (HITACHI, LTD.) 28 September 2017 (2017-09-28) entire text, all drawings | 1-6 |
| A | JP 2021-080118 A (NIKKO RICA CORP.) 27 May 2021 (2021-05-27) entire text, all drawings | 1-6 |

Form PCT/ISA/210 (second sheet) (January 2015)

38

INTERNATIONAL SEARCH REPORT
Information on patent family members

International application No.

**PCT/JP2023/036868**

| Patent document cited in search report | | | Publication date (day/month/year) | Patent family member(s) | | | Publication date (day/month/year) |
|---|---|---|---|---|---|---|---|
| JP | 2008-214158 | A | 18 September 2008 | (Family: none) | | | |
| JP | 62-096310 | A | 02 May 1987 | (Family: none) | | | |
| JP | 61-072709 | A | 14 April 1986 | (Family: none) | | | |
| JP | 2002-114659 | A | 16 April 2002 | (Family: none) | | | |
| WO | 2021/117442 | A1 | 17 June 2021 | (Family: none) | | | |
| JP | 2004-113938 | A | 15 April 2004 | (Family: none) | | | |
| JP | 05-066054 | U1 | 31 August 1993 | (Family: none) | | | |
| JP | 2003-522703 | A | 29 July 2003 | US | 2003/0012720 | A1 | |
| | | | | entire text, all drawings | | | |
| | | | | WO | 2001/058808 | A1 | |
| | | | | EP | 1200345 | A1 | |
| | | | | KR | 10-2000-0024340 | A | |
| | | | | AU | 3420501 | A | |
| | | | | CN | 1362935 | A | |
| CN | 104787770 | A | 22 July 2015 | (Family: none) | | | |
| JP | 2015-504036 | A | 05 February 2015 | US | 2014/0322120 | A1 | |
| | | | | entire text, all drawings | | | |
| | | | | WO | 2013/102414 | A1 | |
| | | | | EP | 2801552 | A1 | |
| | | | | CN | 102653406 | A | |
| | | | | AU | 2012364501 | A | |
| | | | | CA | 2860482 | A | |
| | | | | KR | 10-2014-0116185 | A | |
| | | | | MX | 2014008256 | A | |
| | | | | RU | 2014132182 | A | |
| JP | 2021-038114 | A | 11 March 2021 | (Family: none) | | | |
| JP | 53-092259 | A | 12 August 1978 | (Family: none) | | | |
| JP | 2017-171515 | A | 28 September 2017 | WO | 2017/163873 | A1 | |
| | | | | CN | 108883445 | A | |
| JP | 2021-080118 | A | 27 May 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014181144 A